Europäisches Patentamt

European Patent Office

Office européen des brevets

(11) Publication number: 0 236 977
A2

# EUROPEAN PATENT APPLICATION

(21) Application number: 87103218.1

(22) Date of filing: 06.03.87

(51) Int. Cl.⁴: **C07K 7/10** , A61K 39/15 ,
A61K 39/187 , A61K 39/42 ,
A61K 37/02 , C12N 15/00 ,
G01N 33/53 , C12P 21/02

(30) Priority: 07.03.86 US 837394

(43) Date of publication of application:
16.09.87 Bulletin 87/38

(84) Designated Contracting States:
AT BE CH DE ES FR GB GR IT LI LU NL SE

(71) Applicant: **BIOTECHNOLOGY RESEARCH
PARTNERS, LTD.**
**2450 Bayshore Parkway**
**Mountain View California 94043(US)**

(72) Inventor: **Dale, Beverly**
**195 Ortega Avenue**
**Mountain View Cal. 94040(US)**
Inventor: **White, Robert T.**
**40298 Dolerita Avenue**
**Fremont Cal. 94538(US)**
Inventor: **Cordell, Barbara L.**
**25 Priest Street**
**San Francisco Cal. 94109(US)**

(74) Representative: **Bernhardt, Klaus**
**Radeckestrasse 43**
**D-8000 München 60(DE)**

(54) **Bovine virus diarrhea and hog cholera vaccines.**

(57) Vaccines including recombinant vaccines for immunizing animal hosts against togavirus infection such as bovine viral diarrhea and hog cholera viruses are disclosed. The DNA sequences encoding an immunogenic portion of Togavirus protein are used to generate peptide-containing vaccines carried in vaccinia and to design synthetic peptides which produce antibodies that protect hosts from Togavirus invasion together with methods for their production and use are provided.

EP 0 236 977 A2

## Bovine Virus Diarrhea and Hog Cholera Vaccines

Technical Field

The present invention relates to immunogenic peptides and vaccines for viral infections and, more particularly, to such peptides and vaccinia-based vaccines which find diagnostic and prophylactic use for diseases whose etiological agents are of the family togaviradae, such as bovine virus diarrhea and hog cholera.

Background of the Invention

A fundamental characteristic of living systems is their ability to defend themselves against pathogenic invasion. These defenses can be divided into non-adaptive and adaptive systems, with non-adaptive systems providing a fixed response to any invasion. An example of an advanced non-adaptive defense system involves humoral factors such as lysozyme, C-reactive protein and properdine, which bind to bacterial surfaces and initiate elimination reactions

Of greater interest is the adaptive defense system, possessed by vertebrates, called the immune system, which specifically recognizes and selectively eliminates pathogenic invaders by the immune response. In addition to the specific, adaptive nature of the immune response, the immune system retains a residual memory of previous contacts with foreign pathogens, which enables the system to respond more rapidly to a repeated invasion. This memory component of the immune system is the basis for the pioneering discovery by Jenner of vaccination-induced immunity to smallpox virus.

There has also been widespread use of the specificity of the immune system's antibody-antigen reaction to provide compounds useful for diagnostic assays.

Numerous major veterinary diseases have been discovered having viral etiological agents in the family Togaviridae. These enveloped viruses have single-stranded RNA genomes of approximately 8,000-10,000 nucleotides. Representative members of this family include Semliki forest virus, Sindbis virus and the viral agents which cause bovine virus diarrhea (BVD) and hog cholera. BVD virus (BVDV) and hog cholera virus are closely related immunologically, their membrane proteins showing a high degree of antiserum cross-reactivity.

Bovine virus diarrhea is a disease affecting primarily calves and young cattle. A mild form of BVD characterized by nasal discharge, decreased weight gain, reduced milk production, and mild diarrhea can spread to affect an entire herd. Mucosal disease syndrome, an acute form of BVD, is associated with severe nasal discharge, anorexia, diarrhea, weight loss, high fever and leucopenia. Mucosal disease syndrome can involve 90% mortality in infected cattle.

A significant effect of viral infection in all forms of BVD is the concurrent suppression of the immune system, which can allow opportunistic viral and bacterial infections even in cattle suffering from mild BVD.

There is no known treatment for BVD, and its diagnosis is difficult because its early symptoms are similar to a variety of cattle diseases.

Hog cholera is a highly contagious disease of swine which causes degeneration in the walls of capillaries, resulting in hemorrhages and necrosis of the internal organs. The disease, beginning in a few animals, generally spreads to affect nearly all animals in a herd. Hog cholera is characterized by fever, anorexia, vomiting, discharge from the eyes, and diarrhea. Bacterial infections often accompany the disease in the form of pneumonia and ulcerative enteritis. Most swine die within two weeks of the time symptoms appear, with mortality approaching 100% in some outbreaks.

Modified (attenuated) live virus and killed-virus vaccines have been developed for BVD and hog cholera. The modified live virus vaccine can cause severe reactions or death in inoculated animals. These effects are attributed to inoculation of stressed animals with vaccines contaminated with partially attenuated or virulent virus. The immunosuppressive effects of BVDV infections may also be involved in the problems with modified live vaccines. Use of these vaccines in pregnant cows can cause abortions and fetal anomalies. The killed-virus vaccines are slower to produce immunity, requiring more infections. Following immunization with killed vaccines, dependable immunity does not persist longer than a year.

One alternative to standard killed or modified live vaccine compositions is the use of vaccinia-carried immunogens [derived from BVD virus by recombinant DNA techniques] or of synthetic peptide vaccines designed after the deduced amino acid sequence of antigenic proteins of BVDV. Production of recombinant or synthetic antigens of BVDV or hog cholera virus in the absence of intact Togavirus eliminates the risk of vaccines contaminated with partially attenuated virus and avoids the immunosuppressive effects of the BVDV infection.

Recombinant vaccina virus, expressing foreign antigens can be used as a live (or killed) vaccine and thereby eliminate risk of disease. Mackett, M., et al., J. Virol (1984) 49:857-864 describe this general method. Briefly, vaccinia is a large (187 kb) double-stranded DNA virus which replicates in the cytoplasm of infected cells. It is noninfectious when deproteinized, as it carries its own enzymes for transcription and cannot utilize the machinery of the host cell for this purpose. Vaccinia virus per se was used as the original smallpox vaccine, and is highly desirable as a vaccine carrier because of its low cost and ease of propagation. Freeze-dried vaccinia virus used against smallpox could be mass produced for as little as two cents per dose, while other subunit vaccines, for example, those against hepatitis B, cost approximately $100 per course of immunization. There are other advantages as well. Vaccinia stimulates both the humoral antibody and cell-mediated immunity systems of the subject. The freeze-dried vaccine is stable with refrigeration and is generally potent after a single innoculation. It is also easy to administer under nonsterile field conditions (Smith, G.L., et al., Biotechniques (1984) Nov/Dec:306-312). Because of these advantages, vaccinia has been used as a carrier for antigenic proteins of hepatitis B and herpes simplex (Paoletti, E., et al., Proc Natl Acad Sci (USA) (1984) 81:193-197), rabies (Wiktor, T.J., et al., Proc Natl Acad Sci (USA) - (1984) 81:7194-7198), and human influenza hemagglutinin (Panicali, D., et al., Proc Natl Acad Sci (1983) 80:5364-5368; Smith, G.L., et al., Proc Natl Acad Sci (1983), 80:7155-7159).

Another approach which has reduced risk is the use of synthetic peptides. Knowledge of the amino acid sequence of highly antigenic viral surface proteins presents the opportunity for organic synthesis of short peptides which contain immunogenic determinants. These synthetic peptide vaccines can be used alone, in combination with other subunit vaccines or as primers or boosters for modified live and killed-virus vaccines. Synthetic peptide carrier vaccines have been suggested as a general approach to protection against viral infection (Brown, F., Ann Rev Microbiol (1984) 3:221-235).

One reason these techniques have not been extended to Togaviruses is the absence of sufficient information on the surface proteins of these viruses. This deficiency is remedied by the present invention, which provides genomic and amino acid sequences of a highly antigenic and relevant surface protein characterizing BVDV and hog cholera virus. In the case of this BVDV vaccine a unique opportunity exists, for the same protein or synthetic peptides may be used to immunize swine against hog cholera and cattle against BVD. In addition, the invention provides properly designed vectors and peptides based on these sequences useful in vaccine compositions.

Background Art

The following references contain background of interest to the production of Togavirus vaccines:

Peter, C.P., et al. J. Am. Vet. Asso. (1967) 150:46-52. Thomson, R.G. and Savan, M., Can. J. Comp. Med. Vet. Sci (1963) 27:217-224

Gillespie. J.H. and Timoney, J.F., Hagan Bruner's Infectious Diseases of Animals (1973) Ithaca, Cornell Univ. Press. pp. 665-684

Disclosure of the Invention

It has been found that antibodies which protect animal hosts from the togaviridae can be effectively produced by the administration of BVD viral (BVDV) proteins and peptides which have been prepared in accordance with the present invention.

Such compounds of the present invention useful as diagnostic agents and vaccines also include synthetic analog BVDV peptides. These synthetic peptides can optionally be size enhanced, such as by linkage to carrier proteins, to engender an immune response. Such analog peptide compounds are generally identified by the following formulae:

(I) $X_1$-$AA_1$-$AA_2$-$AA_3$-$AA_4$-$AA_5$-$AA_6$-$AA_7$-$AA_8$-$AA_9$-$AA_{10}$-$AA_{11}$-$AA_{12}$-$AA_{13}$-$AA_{14}$-$AA_{15}$-$AA_{16}$-$AA_{17}$-$AA_{18}$-$AA_{19}$-$AA_{20}$-$AA_{21}$-$AA_{22}$-$AA_{23}$-$AA_{24}$-$AA_{25}$-$AA_{26}$-$AA_{27}$-$Y_1$

wherein:

3

AA₁ is any neutral, polar amino acid residue, preferably S;

AA₂ and AA₂₄ are each any neutral, non-polar amino acid residue, preferably P;

AA₃ and Aa₇ are each any acidic, polar amino acid residue, preferably E;

AA₄ and AA₁₀ are each any neutral, non-polar amino acid residue, preferably M;

AA₅, AA₁₆ and AA₁₉ are each any basic, polar amino acid residue, preferably K;

AA₆ and AA₂₃ are each any neutral, non-polar amino acid residue, preferably L;

AA₈ and AA₁₇ are each any acidic, polar amino acid residue, preferably D;

AA₉, AA₂₁ and AA₂₇ are each any neutral, polar amino acid residue, preferably T;

AA₁₁ is any neutral, non-polar amino acid residue, preferably V;

AA₁₂ is any neutral, non-polar amino acid residue, preferably I;

AA₁₃ is any neutral, non-polar amino acid residue, preferably A;

AA₁₄ is any neutral, non-polar amino acid residue, preferably W;

AA₁₅ and AA₂₆ are each any neutral, polar amino acid residue, preferably C;

AA₁₈ is any neutral, non-polar amino acid residue, preferably G;

AA₂₀ is any neutral, non-polar amino acid residue, preferably F;

AA₂₂ is any neutral, polar amino acid residue, preferably Y; and

AA₂₅ is any basic, polar amino acid residue, preferably R;

and wherein:

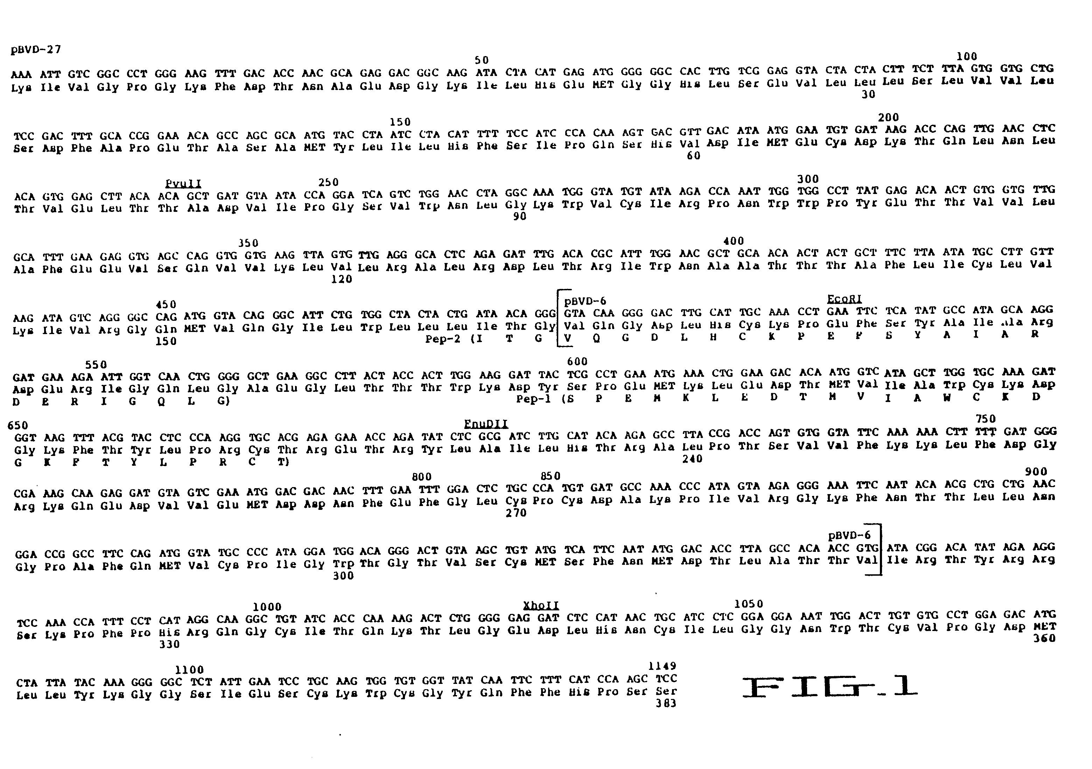

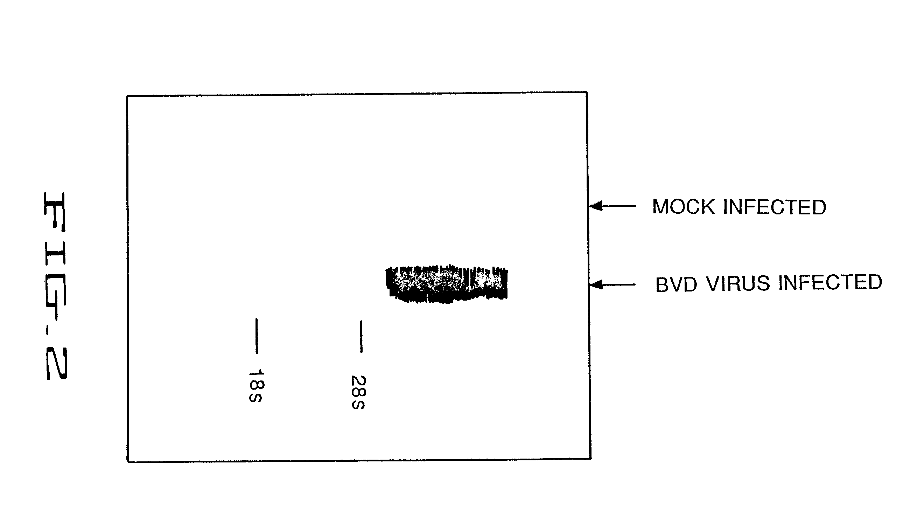

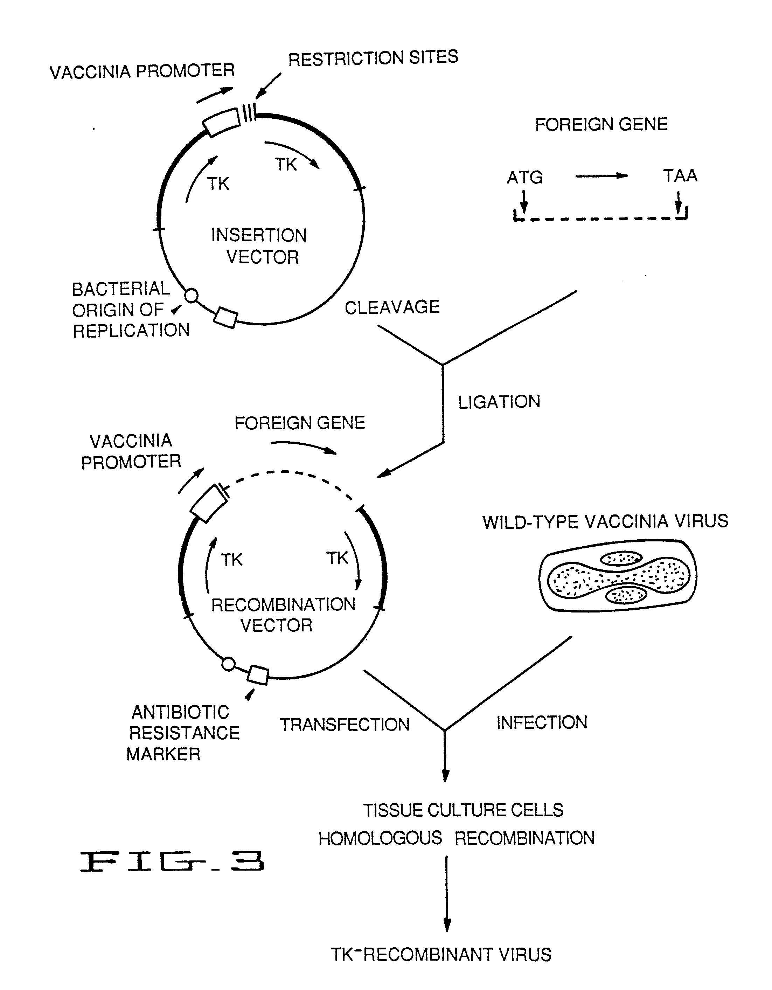

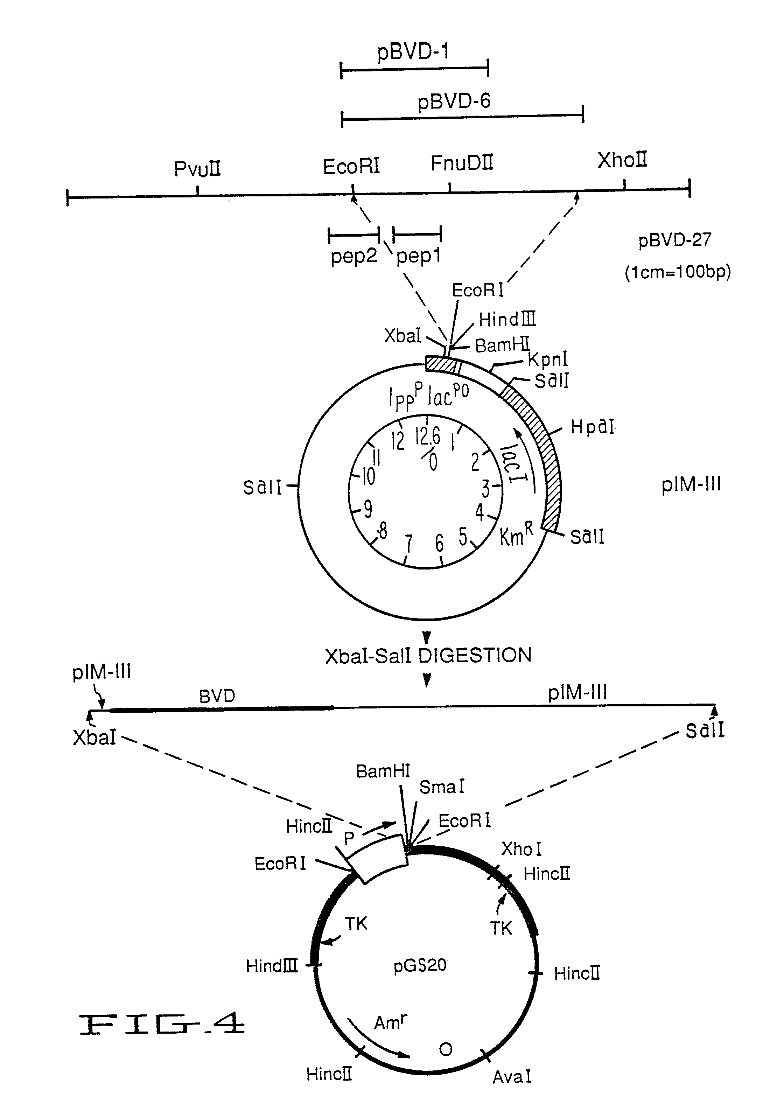

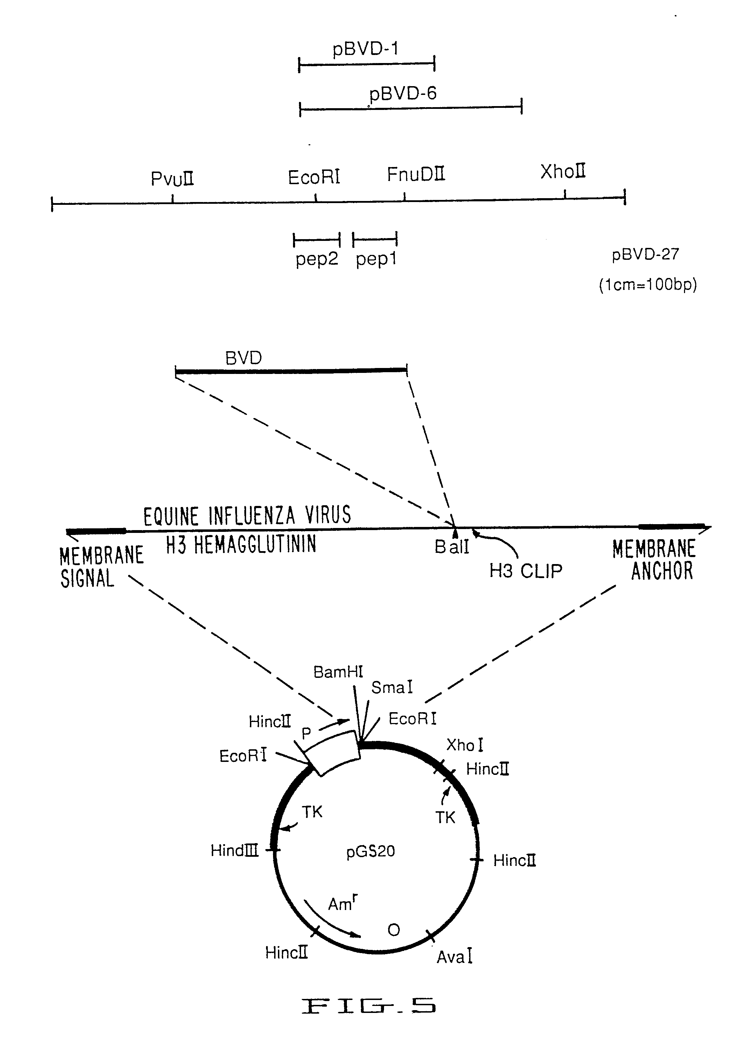

X₁ is hydrogen, amido, acetyl, or additionally includes an amino acid, dipeptide, tripeptide or an oligopeptide of up to 200 amino acid residues, particularly the sequence of residues disclosed as residues 1 to 197 in Figure 1, and including N-acetyl derivatives thereof;

Y₁ is hydroxyl, amido, or an amino acid, dipeptide, tripeptide or an oligopeptide of up to 160 amino acid residues, particularly the sequence of residues disclosed as residues 227 to 383 in Figure 1, including Carboxy-terminal amide derivatives thereof; or

(II)  X₂-aa₁-aa₂-aa₃-aa₄-aa₅-aa₆-aa₇-aa₈-aa₉-aa₁₀-aa₁₁-aa₁₂-aa₁₃-aa₁₄-aa₁₅-aa₁₆-aa₁₇-aa₁₈-aa₁₉-aa₂₀-aa₂₁-aa₂₂-aa₂₃-aa₂₄-aa₂₅-aa₂₆-aa₂₇-aa₂₈-Y₂

wherein:

aa₁, aa₁₈ and aa₂₄ are each any neutral, non-polar amino acid residue, preferably I;

aa₂ is any neutral, polar amino acid residue, preferably T;

aa₃, aa₆, aa₂₅ and aa₂₈ are each any neutral, polar amino acid residue, preferably G;

aa₄ is any neutral, non-polar amino acid residue, preferably V;

aa₅ and aa₂₆ are each any neutral, polar amino acid residue, preferably Q;

aa₇ and aa₂₁ are each any acidic, polar amino acid residue, preferably D;

aa₈ and aa₂₇ are each any neutral, non-polar amino acid residue, preferably L;

aa₉ is any basic, polar amino acid residue, preferably H;

aa₁₀ is any neutral, polar amino acid residue, preferably C;

AA₁₁ is any basic, polar amino acid residue, preferably K;

aa₁₂ is any neutral, non-polar amino acid residue, preferably P;

aa₁₃ and aa₂₂ are each any acidic, polar amino acid residue, preferably E;

aa₁₄ is any neutral, non-polar amino acid residue, preferably F;

AA₁₅ is any neutral, polar amino acid residue, preferably S;

aa₁₆ is any neutral, polar amino acid residue, preferably Y;

aa₁₇ and aa₁₉ are each any neutral, non-polar amino acid residue, preferably A; and

aa₂₀ and aa₂₃ are each any basic, polar amino acid residue, preferably R;

and wherein:

X₂ is hydrogen, amido, acetyl or additionally includes an amino acid, dipeptide, tripeptide or an oligopeptide of up to 160 amino acid residues, particularly the sequence of residues disclosed as residues 1 to 160 in Figure 1, and including N-acetyl derivatives thereof;

Y₂ is hydroxyl, amido, or an amino acid, dipeptide, tripeptide or an oligopeptide of up to 200 amino acid residues, particularly the sequence of residues disclosed as residues 189 to 383 in Figure 1, including Carboxy-terminal amide derivatives thereof;

wherein, for each amino acid residue, or $X_n$ or $Y_n$ having amino acid residues or sequences of residues therein, each residue of the peptide can be the D-isomer of L-isomer; and

each residue having the capability of being substituted with a substituent group, preferably an organic group including any of hydrogen or an aliphatic, aromatic or alkaryl group of from one to ten, usually one to six carbon atoms, including groups having substitutions of three or less nitrogen, oxygen or sulfur atoms as amido, thio or oxy, including hydroxy, thiol and ethers, wherein the ether is usually an alkyl ether, generally of one carbon atom, e.g. methyl.

4

The protein and peptide compounds of the present invention comprise generally a sequence of amino acid residues from a predominantly hydrophilic region of at least one BVDV surface protein. Such compounds are capable, when introduced in an immunogenically effective amount into an animal host, of inducing the host to produce antibodies which recognize and specifically bind to the peptide or protein, the BVD togavirus protein, the Togaviridae, and immunologically related varients of any of these.

Also provided are vaccine compositions containing compounds of the present invention, including the nontoxic addition salts, amides and esters thereof, together with a pharmaceutically acceptable carriers, and physiologically tolerable liquid, gel or solid diluants, adjuvants and excipients. One presently preferred class of vaccine compositions comprises modified vaccinia virus engineered to express the BVD/hog cholera surface peptide of the Togavirus infective agents or a chimeric protein comprising the BVDV peptide and an unrelated surface antigen. Administration of imunogenically effective doses of these compositions to animal hosts can provide effective delivery of the above-recited immunizing reactions to said hosts.

Also provided in the present invention are methods for producing such compounds and compositions, such as chemical synthesis or by employing all or part of the provided genetic sequence encoding the hydrophilic highly antigenic peptide of the BVDV Togavirus protein. Methods for using the compounds and compositions as diagnostic and therapeutic agents are also provided.

Brief Description of the Drawings

In the appended drawings, which form a part of the present disclosure,

Figure 1 displays the nucleotide sequence, and the corresponding sequence of amino acid residues coded thereby, of pBVD-27;

Figure 2 depicts a northern blot analysis of BVDV RNA using BVD-1 cDNA probe;

Figure 3 shows a general method which can be used to prepare recombinant vaccinia capable of expressing the BVDV derived genetic sequences;

Figure 4 portrays the construction of vectors for preparing recombinant vaccinias which code for the expression of compounds of the present invention as chimeric proteins with E. Coli lipoprotein sequences;

Figure 5 portrays the construction of vectors for preparing recombinant vaccinias which code for the expression of compounds of the present invention as chimeric proteins with equine influenza virus hemagglutinin sequences;

Figure 6 portrays the construction of vectors for preparing recombinant vaccinias which code for the expression of compounds of the present invention as chimeric proteins with an alternative equine influenza virus hemagglutinin sequence to that disclosed in Figure 5; and

Figure 7 portrays the construction of vectors for preparing recombinant vaccinias which code for the expression of compounds of the present invention as chimeric proteins with equine influenza virus neuraminidase sequences.

Best Mode of Carrying Out the Invention

In accordance with the present invention, it has been found that antibodies which protect animal hosts from the togaviridae can be effectively produced by the administration of BVD viral (BVDV) peptides which have been prepared in accordance with the present invention.

Virus specific proteins have been examined in cultured cells infected with BVDV (Purchio, A.F., et al., J. Virol (1984) 50;666-669). By using antisera obtained from infected cows, three major viral-specific polypeptides with molecular weights of 115,000, 80,000 and 55,000 were observed. Minor proteins of 45,000 and 38,000 daltons were also noted. The 115,000 and 80,000 polypeptides, but not the 55,000 polypeptide, were shown to be structurally related. The 55,000 protein has also been shown to be glycosylated.

It has been discovered that peptides having antigenic regions derived from the 55,000 dalton BVDV protein can find use as vaccines and substrates for diagnostic assays in accordance with the practice of the present invention. The peptide compounds comprise a sequence of amino acid residues from a region of the BVDV protein. Such compounds are capable, when introduced in an immunogenically effective amount into an animal host, of inducing the host to produce antibodies which recognize and specifically bind to the peptide, and BVD togavirus protein, the BVD virus, the Togaviridae, and immunologically related varients of any of these.

Also in accordance with the present invention, compounds found useful as diagnostic agents and vaccines include synthetic analog BVDV peptides. These synthetic peptides can optionally be size enhanced, such as by linkage to carrier proteins, to engender an immune response. Such analog peptide compounds, examples of which are disclosed in the protein sequence of the present invention, are generally identified by the following formulae:

(I) $X_1$-$AA_1$-$AA_2$-$AA_3$-$AA_4$-$AA_5$-$AA_6$-$AA_7$-$AA_8$-$AA_9$-$AA_{10}$-$AA_{11}$-$AA_{12}$-$AA_{13}$-$AA_{14}$-$AA_{15}$-$AA_{16}$-$AA_{17}$-$AA_{18}$-$AA_{19}$-$AA_{20}$-$AA_{21}$-$AA_{22}$-$AA_{23}$-$AA_{24}$-$AA_{25}$-$AA_{26}$-$AA_{27}$-$Y_1$

wherein:

$AA_1$ is any neutral, polar amino acid residue, preferably S;

$AA_2$ and $AA_{24}$ are each any neutral, non-polar amino acid residue, preferably P;

$AA_3$ and $AA_7$ are each any acidic, polar amino acid residue, preferably E;

$AA_4$ and $AA_{10}$ are each any neutral, non-polar amino acid residue, preferably M;

$AA_5$, $AA_{16}$ and $AA_{19}$ are each any basic, polar amino acid residue, preferably K;

$AA_6$ and $AA_{23}$ are each any neutral, non-polar amino acid residue, preferably L;

$AA_8$ and $AA_{17}$ are each any acidic, polar amino acid residue, preferably D;

$AA_9$, $AA_{21}$ and $AA_{27}$ are each any neutral, polar amino acid residue, preferably T;

$AA_{11}$ is any neutral, non-polar amino acid residue, preferably V;

$AA_{12}$ is any neutral, non-polar amino acid residue, preferably I;

$AA_{13}$ is any neutral, non-polar amino acid residue, preferably A;

$AA_{14}$ is any neutral, non-polar amino acid residue, preferably W;

$AA_{15}$ and $AA_{26}$ are each any neutral, polar amino acid residue, preferably C;

$AA_{18}$ is any neutral, non-polar amino acid residue, preferably G;

$AA_{20}$ is any neutral, non-polar amino acid residue, preferably F;

$AA_{22}$ is any neutral, polar amino acid residue, preferably Y; and

$AA_{25}$ is any basic, polar amino acid residue, preferably R;

and wherein:

$X_1$ is hydrogen, amido, acetyl, or additionally includes an amino acid, dipeptide, tripeptide or an oligopeptide of up to 200 amino acid residues, particularly the sequence of residues disclosed as residues 1 to 197 in Figure 1, and including N-acetyl derivatives thereof;

$Y_1$ is hydroxyl, amido, or an amino acid, dipeptide, tripeptide or an oligopeptide of up to 160 amino acid residues, particularly the sequence of residues disclosed as residues 227 to 383 in Figure 1, including Carboxy-terminal amide derivatives thereof; or

(II) $X_2$-$aa_1$-$aa_2$-$aa_3$-$aa_4$-$aa_5$-$aa_6$-$aa_7$-$aa_8$-$aa_9$-$aa_{10}$-$aa_{11}$-$aa_{12}$-$aa_{13}$-$aa_{14}$-$aa_{15}$-$aa_{16}$-$aa_{17}$-$aa_{18}$-$aa_{19}$-$aa_{20}$-$aa_{21}$-$aa_{22}$-$aa_{23}$-$aa_{24}$-$aa_{25}$-$aa_{26}$-$aa_{27}$-$aa_{28}$-$Y_2$

wherein:

$aa_1$, $aa_{18}$ and $aa_{24}$ are each any neutral, non-polar amino acid residue, preferably I;

$aa_2$ is any neutral, polar amino acid residue, preferably T;

$aa_3$, $aa_6$, $aa_{25}$ and $aa_{28}$ are each any neutral, polar amino acid residue, preferably G;

$aa_4$ is any neutral, non-polar amino acid residue, preferably V;

$aa_5$ and $aa_{26}$ are each any neutral, polar amino acid residue, preferably Q;

$aa_7$ and $aa_{21}$ are each any acidic, polar amino acid residue, preferably D;

$aa_8$ and $aa_{27}$ are each any neutral, non-polar amino acid residue, preferably L;

$aa_9$ is any basic, polar amino acid residue, preferably H;

$aa_{10}$ is any neutral, polar amino acid residue, preferably C;

$AA_{11}$ is any basic, polar amino acid residue, preferably K;

$aa_{12}$ is any neutral, non-polar amino acid residue, preferably P;

$aa_{13}$ and $aa_{22}$ are each any acidic, polar amino acid residue, preferably E;

$aa_{14}$ is any neutral, non-polar amino acid residue, preferably F;

$AA_{15}$ is any neutral, polar amino acid residue, preferably S;

$aa_{16}$ is any neutral, polar amino acid residue, preferably Y;

$aa_{17}$ and $aa_{19}$ are each any neutral, non-polar amino acid residue, preferably A; and

$aa_{20}$ and $aa_{23}$ are each any basic, polar amino acid residue, preferably R;

and wherein:

$X_2$ is hydrogen, amido, acetyl, or additionally includes an amino acid, dipeptide, tripeptide or an oligopeptide of up to 160 amino acid residues, particularly the sequence of residues disclosed as residues 1 to 160 in Figure 1, and including N-acetyl derivatives thereof;

$Y_2$ is hydroxyl, amido, or an amino acid, dipeptide, tripeptide or an oligopeptide of up to 200 amino acid residues, particularly the sequence of residues disclosed as residues 189 to 383 in Figure 1, including Carboxy-terminal amide derivatives thereof;

wherein, for each amino acid residue, or $X_n$ or $Y_n$ having amino acid residues or sequences of residues therein, each residue of the peptide can be the D-isomer or L-isomer; and

each residue having the capability of being substituted with a substituent group, preferably an organic group including any of hydrogen or an aliphatic, aromatic or alkaryl group of from one to ten, usually one to six carbon atoms, including groups having substitutions of three of less nitrogen, oxygen or sulfur atoms as amido, thio or oxy, including hydroxy, thiol and ethers, wherein the ether is usually an alkyl ether, generally of one carbon atom, e.g. methyl.

Neutral nonpolar amino acid residues are taken to mean those residues with hydrophobic side groups at physiologic pH values, generally aliphatic or aromatic hydrocarbons of from zero to ten, usually one to six carbon atoms, which may be substituted with two or less nitrogen, oxygen or sulfur atoms, including such amino acids as Alanine (A), Valine (V), Leucine (L), Isoleucine (I), Proline (P), Methionine (M), Phenylalanine (F) and Tryptophan (W).

Neutral polar amino acid residues are taken to mean those residues with hydrophilic, uncharged side groups at physiologic pH values, including such amino acids as Glycine (G), Serine (S), Threonine (T), Cysteine (C), Tyrosine (Y), Asparagine (N) and Glutamine (Q).

Acidic polar amino acid residues are taken to mean those residues with hydrophilic, negatively charged side groups at physiologic pH values, including such amino acids as Aspartic acid (D) and Glutamic acid - (E).

Basic polar amino acid residues are taken to mean those residues with hydrophilic, positively charged side groups at physiologic pH values, including such amino acids as Lysine (K), Arginine (R) and Histidine - (H).

Certain presently preferred embodiments included in the above formulae include, for example, without showing any disulfide bonds:

$X_1$-S-P-E-M-K-L-E-D-T-M-V-I-A-W-C-K-D-G-K-F-T-Y-L-P-R-C-T-$Y_1$;

wherein $X_1$ and $Y_1$ are as previously defined; and

$X_2$-I-T-G-V-Q-G-D-L-H-C-K-P-E-F-S-Y-A-I-A-R-D-E-R-I-G-Q-L-G-$Y_2$

wherein $X_2$ and $Y_2$ are as previously defined.

The nomenclature used to described protein and peptide compounds of the present invention follows the conventional practice wherein the amino group is presented to the left and the carboxy group to the right of each amino acid residue and each residue is represented by a single letter or three letter designation, corresponding to the trivial name of the amino acid, in accordance with the following schedule:

| Amino Acid | One-letter Symbol | Three-letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

In the present application, the L form of any amino acid residue having an optical isomer is intended unless otherwise expressly indicated.

Compounds within the scope of the present invention can also be obtained by modifying the above recited formulae in numerous ways while preserving the immunogenicity of the compounds thus obtained. For example, while the amino acids of these compounds are normally in the natural L form, one or more, usually two or less and preferably one amino acid may be replaced with the optical isomer D form, or a DL-racemic mixture can be provided in the molecules comprising the peptide compound.

Amino acid residues contained within the compounds can also be modified by amidation, acetylation or substituted with other chemical groups which can, for example, change the solubility of the compounds without effecting their activity. For example, the Carboxy-terminal residue will have a carbonyl carbon which has been substituted with an amino group to form a Carboxy-terminal amido group. In general, the nitrogen of the amido group, covalently bound to the carbonyl carbon, will have two substituent groups, each of which can be hydrogen, alkyl, a benzylic group (substituted or unsubstituted), and any one of which can be a nitrogen containing moiety such as hydrazide and the other can be hydrogen, or either group can be a basic or neutral dipeptide and the other can be hydrogen or an alkyl group. Representative among such amido groups are: $-NH_2$, $-NHCH_3$, $-N(CH_3)_2$, among others. In forming amidated analogs of the present invention, the analog compound can be synthesized directly, for example using BOC-AA-pMBHA-Resin or Boc-AA-BHA-Resin, wherein AA is the selected Carboxy-terminal amino acid of the desired analog compound as described in further detail below. Alternatively, the analog compounds of the present invention can be chemically amidated subsequent to peptide synthesis using means well known to the art, or enzymatically amidated.

In addition, one or more amino acid residues can be replaced by functionally equivalent residues while preserving the immunogenicity of the compounds thus obtained. For example basic polar amino acids can be replaced with other basic polar amino acids and acidic polar amino acids can be replaced with other acidic polar amino acids. However, more preferred replacements include the substitution of alternative non-polar amino acid residues for those residues specifically disclosed.

The compounds of the present invention can also be modified by extending, decreasing or substituting in the compounds' amino acid sequence, e.g., by the addition or deletion of amino acids or oligopeptides on either the Amino-terminal or Carboxy-terminal end, or both, of the sequences disclosed above. Particularly, $Y_n$ can be amide or an amino acid or oligopeptide of not more than about 200, more usually 80, and desirably 50 or less amino acids and $X_n$ can be N-acetyl or an amino acid or oligopeptide of not more than about 200, and desirably up to about 100 amino acids, provided the modifications do not adversely effect the immunogenic activities of the subject compounds.

Amino acid deletions can provide shortened immunogenic BVDV peptide sequences within the general formulae I and II disclosed above. In particular, formula I can represent an immunogenic, BVDV-specific peptide where $AA_{1-14}$ and $AA_{27}$ can be deleted and provide the peptide of the formula:

(IA) $X_{1A}$-$AA_{15}$-$AA_{16}$-$AA_{17}$-$AA_{18}$-$AA_{19}$-$AA_{20}$-$AA_{21}$-$AA_{22}$-$AA_{23}$-$AA_{24}$-$AA_{25}$-$AA_{26}$-$Y_{1A}$

wherein:

$AA_{15}$ and $AA_{26}$ are each any neutral, polar amino acid residue, preferably C;

$AA_{16}$ and $AA_{19}$ are each any basic, polar amino acid residue, preferably K;

$AA_{17}$ is any acidic, polar amino acid residue, preferably D;

$AA_{18}$ is any neutral, non-polar amino acid residue, preferably G;

$AA_{20}$ is any neutral, non-polar amino acid residue, preferably F;

$AA_{21}$ is any neutral, polar amino acid residue, preferably T;

$AA_{22}$ is any neutral, polar amino acid residue, preferably Y; and

$AA_{23}$ is any neutral, non-polar amino acid residue, preferably L;

$AA_{24}$ is any neutral, non-polar amino acid residue, preferably P; and

$AA_{25}$ is any basic, polar amino acid residue, preferably R;

and wherein:

$X_{1A}$ is hydrogen, amido, acetyl, or additionally includes an amino acid, dipeptide, tripeptide or an oligopeptide of up to 220 amino acid residues, particularly the sequence of residues disclosed as residues 1 to 213 in Figure 1, and including N-acetyl derivatives thereof; and

$Y_{1A}$ is hydroxyl, amido, or an amino acid, dipeptide, tripeptide or an oligopeptide of up to 160 amino acid residues, particularly the sequence of residues disclosed as residues 226 to 383 in Figure 1, including Carboxy-terminal amide derivatives thereof.

In addition, amino acid deletions can provide shortened immunogenic BVDV peptide sequences within the general formula II disclosed above. In particular, formula II can represent two immunogenic, BVDV-specific peptides where $aa_{1-2}$ and $aa_{12}$ through $aa_{28}$ can be deleted or $aa_1$ through $aa_{19}$ can be deleted and provide the peptides of the formulae:

(IIA) $X_{2A}$ -$aa_3$-$aa_4$-$aa_5$-$aa_6$-$aa_7$-$aa_8$-$aa_9$-$aa_{10}$-$aa_{11}$-$Y_{2A}$

wherein:

$aa_3$ and $aa_6$ are each any neutral, polar amino acid residue, preferably G;

$aa_4$ is any neutral, non-polar amino acid residue, preferably V;

$aa_5$ is any neutral, polar amino acid residue, preferably Q;

$aa_7$ is any acidic, polar amino acid residue, preferably D;

$aa_8$ is any neutral, non-polar amino acid residue, preferably L;

$aa_9$ is any basic, polar amino acid residue, preferably H;

$aa_{10}$ is any neutral, polar amino acid residue, preferably C; and

$AA_{11}$ is any basic, polar amino acid residue, preferably K;

and wherein:

$X_{2A}$ is hydrogen, amido, acetyl, or additionally includes an amino acid, dipeptide, tripeptide or an oligopeptide of up to 165 amino acid residues, particularly the sequence of residues disclosed as residues 1 to 162 in Figure 1, and including N-acetyl derivatives thereof; and

$Y_{2A}$ is hydroxyl, amido, or an amino acid, dipeptide, tripeptide or an oligopeptide of up to 220 amino acid residues, particularly the sequence of residues disclosed as residues 172 to 383 in Figure 1, including Carboxy-terminal amide derivatives thereof; or

(IIB) $X_{2B}$-$aa_{20}$-$aa_{21}$-$aa_{22}$-$aa_{23}$-$aa_{24}$-$aa_{25}$-$aa_{26}$-$aa_{27}$-$aa_{28}$-$Y_{2B}$

wherein:

$aa_{20}$ and $aa_{23}$ are each any basic, polar amino acid residue, preferably R;

9

$aa_{21}$ is any acidic, polar amino acid residue, preferably D;

$aa_{22}$ is any acidic, polar amino acid residue, preferably E;

$aa_{24}$ is any neutral, non-polar amino acid residue, preferably I;

$aa_{25}$ and $aa_{28}$ are each any neutral, polar amino acid residue, preferably G;

$aa_{26}$ is any neutral, polar amino acid residue, preferably Q; and

$aa_{27}$ is any neutral, non-polar amino acid residue, preferably L;

and wherein:

$X_{2B}$ is hydrogen, amido, acetyl, or additionally includes an amino acid, dipeptide, tripeptide or an oligopeptide of up to 180 amino acid residues, particularly the sequence of residues disclosed as residues 1 to 179 in Figure 1, and including N-acetyl derivatives thereof;

$Y_{2B}$ is hydroxyl, amido, or an amino acid, dipeptide, tripeptide or an oligopeptide of up to 200 amino acid residues, particularly the sequence of residues disclosed as residues 189 to 383 in Figure 1, including Carboxy-terminal amino derivatives thereof.

Certain presently preferred embodiments included in the above formulae include, for example, without showing any disulfide bonds:

$X_{1A}$-C-K-D-G-K-F-T-Y-L-P-R-C-$Y_{1B}$;

wherein $X_{1A}$ and $Y_{1A}$ are as previously defined; and

$X_{2A}$-G-V-Q-G-D-L-H-C-K-$Y_{2A}$; and

$X_{2B}$-R-D-E-R-I-G-Q-L-G-$Y_{2B}$

wherein $X_{2A}$, $X_{2B}$, $Y_{2A}$ and $Y_{2B}$ are as previously defined.

The present compounds can also be modified by altering the order in which certain residues are provided in the amino acid sequence of the compound. In particular, it will be readily appreciated that, while certain sequences of oligopeptides are provided as preferred embodiments for $X_1$, $X_{1A}$, $X_2$, $X_{2A}$, $X_{2B}$, $Y_1$, $Y_{1A}$, $Y_2$, $Y_{2A}$, and $Y_{2B}$, these amino acid residues have been shown not to be essential to the immunogenic activity of the compounds.

Also provided in the present invention are methods for producing such compounds and compositions, such as chemical synthesis and by employing all or part of the provided genetic sequence encoding the hydrophilic highly antigenic peptide of the Togavirus protein.

Numerous compounds within the scope of the present invention can be synthesized chemically by means well-known in the art such as, e.g., solid phase peptide synthesis. The synthesis is commenced from the Carboxy-terminal end of the peptide using an alpha-amino protected amino acid. t-Butyloxycarbonyl - (Boc) protective groups can be used for all amino groups even though other protective groups are suitable. For example, Boc-G-OH or Boc-T-OH (i.e., selected Carboxy-terminal amino acids) can be esterified to chloromethylated polystyrene resin supports. The polystyrene resin support is preferably a copolymer of styrene with about 0.5 to 2% divinyl benzene as a cross-linking agent which causes the polystyrene polymer to be completely insoluble in certain organic solvents. See Stewart et al., Solid-Phase Peptide Synthesis, W. H. Freeman Co., San Francisco (1969) and Merrifield, J. Am. Chem. Soc. 85:2149-2154 - (1963). These and other methods of peptide synthesis are also exemplified by U.S. Patent Nos. 3,862,925, 3,842,067, 3,972,859 and 4,105,602.

It will be readily appreciated by those having ordinary skill in the art of peptide synthesis that the intermediates which are constructed in accordance with the present disclosure during the course of synthesizing the present compounds are themselves novel and useful compounds and are thus within the scope of the invention.

Conveniently, compounds may be synthesized using manual techniques or automatically employing, for example, an Applied BioSystems 430A Peptide Synthesizer (Foster City, California) or a Biosearch SAM II automatic peptide synthesizer (Biosearch, Inc. San Rafael, California), following the instructions provided in the instruction manual supplied by the manufacturer.

Alternatively, compounds of the present invention can be produced by expression of the recombinant DNA constructs prepared in accordance with well-known methods. Such production can be desirable to provide large quantities or alternative embodiments of such compounds.

More particularly, modifications in the amino acid sequence of the various compounds to provide alternative embodiments of the present invention can be effected by various changes in the nucleotide sequence of the cloned or synthetic structural gene used to direct the synthesis of the compounds. Included within such modification of the DNA sequence are the replacement of various codons with other codons which, due to the degeneracy of the genetic code, direct the synthesis of the same amino acid.

In addition, by codon substitution, one or more amino acid residues can be replaced by functionally equivalent residues, as disclosed above.

Also provided are vaccine compositions containing compounds of the present invention, including the nontoxic addition salts, amides and esters thereof, together with a pharmaceutically acceptable carriers, and physiologically tolerable liquid, gel or solid diluants, adjuvants and excipients.

One class of vaccine compositions comprises modified vaccinia engineered to express the BVD/hog cholera surface peptide of the Togavirus infective agents or a chimeric protein composed of the BVDV peptide and an unrelated surface antigen. Administration of immunogenically effective doses of these compositions to animal hosts can provide effective delivery of the above-recited immunizing reactions to said hosts.

Furthermore, compounds of the present invention can be used in straight chain or cyclic ring form, to provide vaccine compositions. The present compounds can also be polymerized, co-polymerized, mixed with, bonded to, or conjugated or complexed with compounds having the same or similar immunogenic activities to obtain or increase the benefits of vaccine compositions of the present invention. A variety of techniques is available in the art for such polymerization or conjugation. The most commonly used polymerization technique employs glutaraldehyde and effects self-condensation of the peptide. Alternatively, adjacent peptide repeating units can be bonded together by oxidized cysteine residues or crosslinkers, to provide polymer or co-polymer compounds of the invention. If necessary, additional cysteine residues can be included in the peptide compound to provide for such means of bonding. However, alternative methods of polymerization or co-polymerization are well known in the art.

For small peptide compounds, conjugation to a carrier protein is, generally, a preferred approach. Such conjugation is effected by linking agents which attach to each member of the conjugate. For example, hetero bifunctional agents which generate a disulfide link with one component and a peptide link with the other may be employed in the present invention. Any of the synthetic peptides disclosed herein may be prepared with an additional cysteine to permit this mode of linkage. The most popular such linking agent is N-succinidyl-3-(2-pyridlthio) propionate (SPDP). Other such agents, which create a disulfide linkage at one end and an amide linkage through an epsilon-amino on a lysine at the other end, are available. See, for example, Immun. Rev. (1982) 82:185. Still other bifunctional coupling agents form a thioether rather than a disulfide linkage. Many of these thioether-forming agents are commercially available, and include reactive esters of 6-maleimidocaproic acid, 2-bromacetic acid, 2-iodoacetic acid, and the like. Carboxyl groups of these acids can be activated by combining them with succinimide or 1-hydroxy-2-nitro-4-sulfonic acid, sodium salt. In addition, reagents which operate through dehydration, such as dicyclohexylcarbodiimide, can be used.

Selected vaccine compositions will include carrier proteins, which can be any of numerous carrier proteins such as keyhole limpet hemocyanin (KLH), tetnus toxoid, bovine serum albumin, ovalbumin, diptheria toxoid, purified protein derivative (PPD) and the like, to which an immunogenic peptide of the present invention is linked. When introduced in an effective amount as a vaccine into an animal host, such vaccine compositions are typically capable of inducing production of antibodies in the host that immunoreact with the related togavirus and protect the host from infection caused by that togavirus.

As a vaccine, the present invention comprises an effective amount if a peptide antigen which may, alone, serve as the vaccine when present with a physiologically acceptable diluant such as water or saline.

The vaccines of the invention which are recombined vaccinia virus are employed in the conventional manner. Vaccinia virus can be cultured in standard media, as described below, or by infecting live animals and harvesting the virus from the lesions. The administration of the vaccinia is generally by means of scratching the skin of the host, and non-sterile conditions are often acceptable.

For vaccines which usually contain peptides or proteins as the active ingredient, administration is slightly more complex. Typically, such vaccines, and, optionally, vaccinia vaccines as well, are prepared as injectables, either as liquid solutions or suspensions; solid forms suitable for solution in, or suspension in, liquid prior to injection may also be prepared. The preparation may also be emulsified. The active immunogenic ingredient is often mixed with diluants or excipients which are physiological tolerable and compatible with the active ingredient. Suitable diluants and excipients are, for example, water, saline, dextrose, glycerol, ethanol, or the like, and combinations thereof. In addition, if desired the vaccine may contain minor amounts of auxiliary substances such as wetting or emulsifying agents, pH buffering agents, or adjuvants which enhance the effectiveness of the vaccine. Such adjuvants include, for example, complete Freund's adjuvant (CFA), incomplete Freund's adjuvant (IFA) and aluminum hydroxide, among others.

The vaccines are conventionally administered parenterally, by injection, for example, either subcutaneously or intramuscularly. Additional formulations which are suitable for other modes of administration include suppositories, intranasal aerosols, and, in some cases, oral formulations. For suppositories, traditional binders and excipients may include, for example, polyalkylene glycols or triglycerides; such suppositories may be formed from mixtures containing the active ingredient in the range of 0.5% to 10% preferably 1%-2%. Oral formulations include such normally employed excipients as, for example, pharmaceutical grades of manitol, lactose, starch, magnesium stearate, sodium saccharin, cellulose, magnesium carbonate, and the like. These compositions take the form of solutions, suspensions, tablets, pills, capsules, sustained release formulations, or powders, and contain 10%-95% of active ingredient, preferably 25%-70%.

The peptides may be formulated into the vaccine as neutral or salt forms. Pharmaceutically acceptable non-toxic salts include the acid addition salts (formed with the free amino groups) and which are formed with inorganic acids such as, for example, hydrochloric or phosphoric acids, or such organic acids such as, for example, hydrochloric or phosphoric acids, or such organic acids as acetic, oxalic, tartaric, mandelic, and the like. Salts formed with the free carboxyl groups may be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The peptide or protein-based vaccines are administered in a manner compatible with the dosage formulation, and in such amount as will be therapeutically effective and immunogenic. The quantity to be administered depends on the subject host, e.g., cow or hog, to be treated, the capacity of the subject's immune system to synthesize antibodies, and the degree of protection desired. Precise amounts of active ingredient required to be administered are usually particular to each subject, but can be routinely determined. However, suitable dosage ranges are of the order of several hundred micrograms active ingredient per subject.

Suitable regimes for initial administration and booster shots are also variable, but are typified by an initial administration followed in three to four week intervals by a subsequent injection or other administration. Alternately, primer doses of the vaccines of the invention may be used three to four weeks before injection with conventional killed virus vaccine. Booster doses may be used three to four weeks after a primary injection of killed virus vaccine.

A preferred use of the synthetic peptide immunogens is that they be incorporated into existing immunization regimens as either a first or "priming" inoculation or as second and subsequent "boosting" inoculations in combination with other vaccines. It has been shown by others that a single dose of a synthetic peptide "primes" the immune system such that subsequent inoculation with the whole virus (e.g., polio virus) elicits extremely high titers of neutralizing antibodies. The practice of priming or boosting with synthetic peptides offers major advantages: it lessens the number of whole virus vaccine inoculations (and the accompanying side effects) required to achieve immunity, and it retains at least one dose of whole virus vaccine in the immunization regimen thus allowing the animal's immune system an opportunity to see the entire antigenic repertoire of the pathogen.

Compounds of the present invention are shown to have immunogenic activity in animal hosts. Furthermore, compounds of the present invention including synthetic compounds, can provide substrates for diagnostic assays and can find use as intermediates in the production of antisera and antibodies, including monoclonal antibodies, which are useful, e.g., in formulating kits for diagnostic purposes. For example, such compounds can be used in immunoassays where either of the present synthetic peptides, or antibodies capable of recognizing and specifically binding to the present synthetic peptides are conjugated to a label. Labels which have found use in such assays include radioactive isotopes, for example, tritium or $^{125}I$, fluorescers, enzymes, co-enzymes, or the like. Such assay methods and methods for conjugating labels to synthetic peptides or antibodies are well established in the prior art.

The monoclonal antibodies of the present invention can be prepared by fusing mammalian lymphocytes, which have been obtained from the spleens of mammals immunized by peptides of the invention, with a drug-resistant myeloma cell line in the presence of polyethylene glycol. Fusion hybrids are cultured in hypoxanthine/aminopterin/thymidine (HAT) selective medium and colonies are isolated whose supernatant fluids contain antibody specifically reactive with the peptides, the BVDV protein or the togavirus. Monoclonal antibodies can then be prepared in quantity by injecting hybridoma cells intra-peritoneally into mice primed with Pristane. Thereafter ascites fluid containing monoclonal antibodies is recovered from the abdominal cavity of the mouse.

The lymphocytes used in the present invention can be derived from the spleen of any host mammal, such as for example rodents. The host can be sensitized by injection of the isolated peptide or with a vaccine composition as disclosed previously. The initial immunization is generally followed by a series of booster injections and subsequent isolation of the spleen.

Antibodies and peptides of the subject invention may be labeled with any known label for use in diagnostic immunoassays or in any procedure which requires precise discrimination of one organism from another. Known labels include fluorescent compounds, enzymes, chemiluminescent compounds, radioisotopes, and particles including, for example, polymerized beads and magnetic particles, among others. For any chosen label, binding of the antigen or antibody provides for the detection and measurement of the complementary composition.

There are numerous immunoassay methods which employ different labels bound to antibodies or antigens. These can be heterogeneous or homogeneous and can include, for example, enzyme-linked immunosorbent assays (ELISA), radioimmunoassays (RIA), and sandwich assays, among others.

These immunoassay methods can include the addition of a surfactant or solubilizing reagent which can, e.g., expose antigenic determinants which are not readily accessible to the labeled peptides or antibodies. These solubilizing reagents are well known in the art and can include, e.g., Triton®X-100, sodium dodecyl sulfate (SDS), and Nonidet P-40 (NP-40), among others.

The antibodies of the subject invention specifically recognize and bind to an antigenic determinant of the present invention, common to at least BVD and hog cholera virus, and are not substantially cross reactive with non-togavirus pathogens. Having identified the togavirus peptide antigen by these criteria, it is possible to produce other antibodies which will be reactive with the same antigen and hence cross react with BVDV, hog cholera virus and the togavirus.

Methods of the subject invention is useful for the detection and diagnosis of BVD, hog cholera and other togaviral infections. For example, a sample suspected of containing said virus or viral-specific antibodies is reacted with a labeled antibody or peptide antigen probe. After washing to remove unbound, labeled probe, the signal emitted by the label is detected and quantified using appropriate means. The sample is typically of biological origin including such specimens as sputum, lung tissue, blood or urine. The subject methods also can be used to confirm the identity of the virus of interest in culture.

EXPERIMENTAL

Based on the genomic information provided by the invention, numerous general approaches to preparing vaccines can be used. As a representative example, a portion of a BVDV cDNA clone is recombined into a nonessential region of vaccinia virus, which then expresses the inserted BVDV antigen coding sequences. A second approach is to insert a portion of the BVDV cDNA clone, in-frame for proper translation, into another gene encoding a cell surface antigen such as, e.g., influenza virus hemagglutinin or neuraminidase, vesicular stomatitus virus G protein, surface immunoglobulin, herpes simplex virus glycoprotein D, or rabies virus glycoprotein. The chimeric gene is recombined into a non-essential region of vaccinia virus which then expresses the chimeric protein on its surface. These recombinant vaccinias are then employed as BVD/hog cholera vaccines.

In a third approach, synthetic peptides designed from deduced amino acid sequences of the cloned BVDV cDNA, if necessary made immunogenic by size enhancement, are used to prepared vaccines. These vaccines may also be used to boost the protective immune response from animals previously given a single dose of killed or modified whole virus vaccines. Alternatively, the synthetic peptide vaccines may be given as a primer prior to the inoculation with whole virus. In a fourth approach, the synthetic peptides may be given simultaneously with the killed or modified whole virus vaccines as an adjuvant.

A.1. Cloning and Sequencing of the BVD Viral Genetic Material

The BVDV genome consists of a single RNA strand of approximately 10,000-12,000 nucleotides. The manner in which the viral genes are encoded on the genomic RNA is unknown, although it is probably analogous to Picornaviruses whereby a single polypeptide is generated from the RNA which is cleaved post-translationally to produce the various viral proteins. Therefore, cDNA libraries were prepared from total viral RNA using random primers and conventional methods. The libraries were constructed so as to allow potential expression of the random pieces of cDNA representing portions of the entire viral genome. Antisera from BVDV-infected cows were used to screen the libraries using conventional procedures. The antibody-positive reacting cDNA clones were then sequenced and the encoded amino acid sequence of each clone deduced. The deduced amino acid sequences were analyzed for antigen sites using secondary

13

structure computer profiles, and by localizing solvent surfaces of the protein which may be important in the disease process. The identity of the clones as derived from BVDV sequences was determined by hybridization of the cDNA clones specifically to BVDV-infected cell RNA and not to uninfected cell RNA.

## A.2. Generation of Recombinant BVDV-Vaccinia Viruses

The parameters for effecting the integration of foreign DNA into vaccinia are known in the art and are currently in a position for practical utilization.

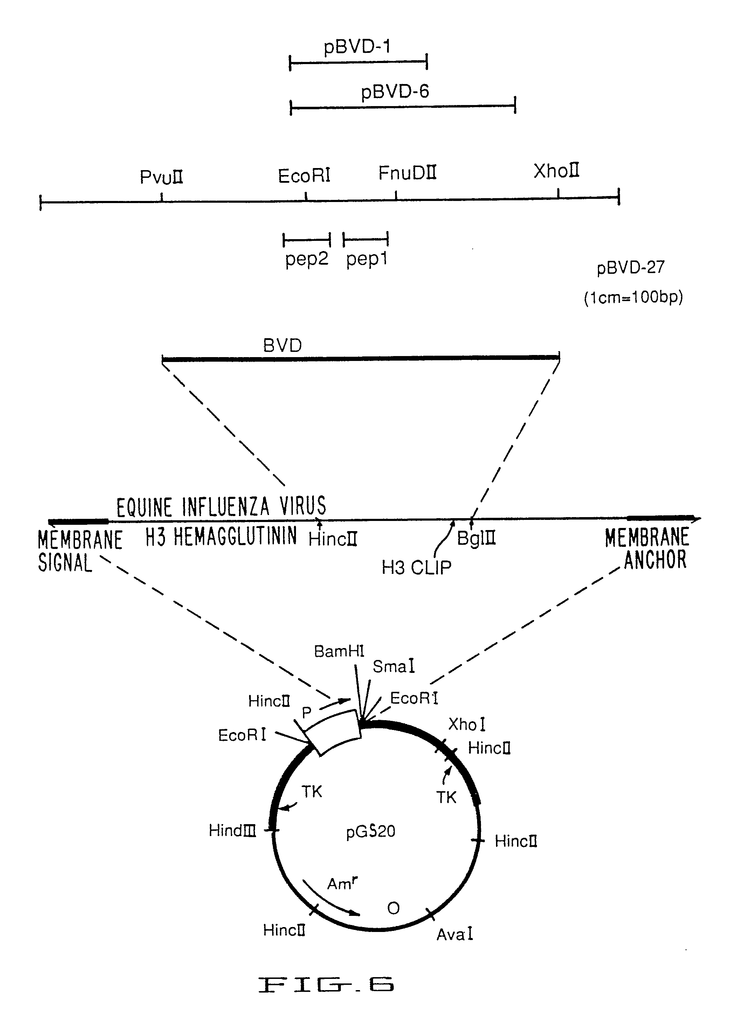

Briefly, the desired immunogen is transferred into a nonessential portion of the vaccinia genome by coninfecting host cells with both native vaccinia and a carrier plasmid which contains the foreign gene sandwiched between sequences homologous with the selected nonessential portion of the vaccinia genome. In addition, the foreign gene is provided with a vaccinia promoter which will permit its expression under the influence of the vaccinia transcription and translation systems. A general purpose vector capable of housing the foreign gene has been disclosed by Moss, B., et al., (Proc. Natl. Acad. Sci. (USA) (1983) 80:7155-7159). A diagram of this vector, pGS20, and its manner of use to obtain a recombinant vaccinia virus containing a desired foreign gene is shown in Figure 3.

pGS20 has a vector fragment derived from pBR328, a vector compatible with E. Coli which contains the E. coli origin of replication and the ampicillin resistance (Amp^R) gene. The vector contains the promoter from the 7.5 k gene of vaccinia (a gene encoding a 7500 dalton protein), excised as a 275 bp HincII/RsaI fragment (Venkatsen, et al., Cell (1981) 25:805-813), which is translocated into the EcoRI site of the vaccinia thymidine kinase (TK) gene. Other restriction site modifications have been made for convenience, and there are BamHI and SmaI restriction sites immediately downstream of the promoter to permit foreign gene cloning (Mackett, M., et al., supra, incorporated herein by reference).

In preparing the vectors of the invention, the DNA encoding the desired immunogen is inserted into pGS20 using the restriction sites downstream of the promoter. In the present invention this DNA is derived from (1) the BVDV cDNA, (2) a BVDV-E. Coli lipoprotein gene fusion, (3) two equine influenza virus hemagglutinin-BVDV gene fusions, and (4) an equine influenza virus neuraminidase-BVDV gene fusion, and comprises an immunologically effective portion thereof. The recombination vector is amplified in E. Coli using transformation to Amp^R and then coinfected along with wild-type vaccinia into CV-1 cells. Unlike other large DNA animal viruses, vaccinia transcribes and replicates its genome in the cytoplasm of infected cells. Many of the enzymes involved in its nucleic acid metabolism, such as DNA and RNA polymerases, enzymes to cap, methylate, and polyadenylate RNA, as well as thymidine kinase, are encoded in its own genome. Indeed, protein-free vaccinia DNA is noninfective. Since it encodes and carries in its own transcriptase and apparently cannot use the transcriptase used by its eukaryotic host, it cannot synthesize required proteins using its DNA alone in combination with host cell machinery. Cells having been transformed with both the recombinant, for example pGS20-derived, vector and vaccinia virus mediate recombination of the DNA portion contained within the vaccinia-corresponding segments of the vector into the vaccinia genome. Use of the TK-encoding sequences to effect recombination is particularly desirable, as not only is this a nonessential portion of the vaccinia genome, but a selectable marker is provided for cells containing the recombined vaccinia--i.e., when the foreign DNA is inserted, the TK gene is inactivated, and the tk-recombinant viruses can be selected by plaque assay on tk-cells in the presence of 5-bromodeoxyuridine (BudR). As shown in Figure 3, tk-recombinant virus plaques can be selected yielding the desired recombinant vaccinia. Additionally, tk-vaccinia have been shown to be $10^5$ to $10^6$ times less virulent in laboratory animals as compared to wild-type vaccinia, indicating the potential for an increased safety factor in animal hosts such as cattle or hogs (Smith, G.L., and Moss, B., Biotechniques (1984) 306-312).

While pGS20 is a convenient illustrative vector, it is understood that alternative constructions involving other vaccinia promoters in other nonessential regions of the gene may be used (Moss, et al., Gene Amplification Analysis, Vol. III, Pappas, T. K., et al., eds. (1982) New York Elsevier. pp. 201-213; Mackett, M., et al., supra). Not only CV-1, but any competent cells susceptible to vaccinia infection can be used to effect the recombination and subsequent propagation of recombinant vaccinias.

## B.3 Preparation of Synthetic Peptide Vaccines

Peptides were identified as representing potential antigenic regions of the 55,000 Dalton BVDV protein by analysis of the predicted secondary structure of the viral protein segment translated from the cloned cDNA. These potential antigenic determinants were synthesized using the solid-state Merrifield method as previously described. As these peptides were often too small to be immunogenic alone, their size would be enhanced, usually by conjugation to a carrier protein, as previously disclosed.

In order to illustrate the present invention more clearly, without implying any limitations, the following examples are provided.

## EXAMPLES

A. Identification of cloned BVDV cDNA sequences encoding an antigen recognized by anti-BVDV and anti-hog cholera virus serum.

### 1. Preparation of BVD Viral cDNA.

BVD-Singer virus was grown in primary cultures of bovine testicle cells in modified Eagle's medium - (MEM) with 10% sheep serum. The cell supernatants were harvested and the virions purified by centrifugation (30,000 xg) onto a 50% sucrose cushion in 0.1 M NaCl, 10 mM Tris-HCl pH 7.5, 1 mM EDTA.

The virions were treated for 30 minutes at 22°C with 10 units RNAse-free DNAse I (Worthington) in 10 mM MgCl₂, 20 mM Tris-HCl, pH 7.5. Virion RNA was extracted with an equal volume of phenol:chloroform, then precipitated with two volumes of 70% ethanol at -20°C.

Prior to cDNA synthesis, the RNA was boiled for one minute, then chilled on ice. First strand cDNA synthesis was performed using an RNA concentration of 5 µg/ml in 100 µl of 100 mM KCl, 50 mM Tris, pH 7.6, 30 mM B-mercaptoethanol, 10 mM MgCl₂, 100 µM of each dNTP, and 200 µg/ml bovine serum albumin (BSA). cDNA synthesis was initiated using 20 µg/ml short (approximately 10-50 nucleotides) random DNA primers by incubation for one hours at 42°C in the presence of 40 units of RNAse-free avian myeloblastosis virus reverse transcriptase. The resulting single-stranded cDNAs averaged 500 nucleotides in length, as determined by alkaline-agarose gel electrophoresis of ³²P-labeled cDNA.

To synthesize a second cDNA strand, the single-strand cDNA was boiled for three minutes and centrifuged to remove denatured protein, diluted 1:1 with water and incubated overnight at 15°C with 50 units E. coli DNA polymerase (Klenow fragment). The second-strand reaction produced cDNA about twice the length of the first strand. The double-strand cDNA was extracted with phenol:chloroform and precipitated with ethanol as disclosed previously.

The double-stranded cDNA was digested with 600 units of S1 nuclease in 300 mM NaCl, 30 mM NaOAc pH 4.5, 3mM ZnCl₂ for 30 minutes at 37°C The cDNA was extracted with phenol:chloroform as above, and small oligonucleotides were removed by ethanol precipitation as follows: one-half volume of 7.5 M ammonium acetate and two volumes ethanol were added to the cDNA solution, which was then stored at -70°C for 15 minutes. This procedure was repeated twice.

Oligo-dC tails were added to the cDNA in a reaction containing 0.2 M potassium cacodylate, 25 mM Tris, pH 6.9, 2 mM dithiothreitol, 0.5 mM CoCl₂, 200 µM dCTP, 400 µg/ml BSA, and 40 units calf thymus terminal deoxynucleotide transferase for 5 minutes at 22°C. The reaction was extracted with phenol:chloroform, and small oligonucleotides were removed with three ethanol precipitations in the presence of ammonium acetate as above.

One µg of pBR322, which has been cleaved with PstI within the B-lactamase gene and tailed with oligo dG, was annealed with the deoxy(C)-tailed cDNA at a vector concentration of 5 µg/ml. The hybrid vectors were used to transform E. coli K12 MC1061 and screened for tetracycline resistance. The resulting library of tetracycline resistant recombinant clones contained 2500 transformants, of which 95% contained plasmids with cDNA inserts.

Recombinant clones were screened for expression of BVDV antigens by an antibody colony screening procedure as described by Helfman et al., Proc. Natl. Acad. Sci. USA 80:31-35 (1983). The recombinant library was plated out and duplicate replicas of the colonies were made on nitrocellulose filters. The colonies were lysed with chloroform vapor for 20 minutes. The filters were then incubated overnight at room temperature in 50 mM Tris, pH 7.5, 150 mM NaCl, 5 mM MgCl₂, 3% BSA, 1 µg/ml DNAse I and 40 µg/ml lysozyme. The filters were rinsed briefly in Tris-saline (50 mM Tris, pH 7.5, 150 mM NaCl) and incubated for one hour at room temperature in Tris-saline containing 3% BSA and a 1:20 dilution of calf serum

The four pGS20 constructs are described as follows and are illustrated in Figures 4 through 7. In each Figure, a schematic representation of pBVD27 appears at the top. The location of the original immunoreactive clones (pBVD-6 and pBVD-1), the synthetic peptides (pep 1 and pep-2), and restriction sites used for pGS20 cloning are all described. (1) The first pGS20-BVD construct is a chimera from a previous construct which expresses a BVDV-immunoprecipitable antigen in bacteria. In that construct, a 450 bp BVDV cDNA originating at the Eco RI site and extending to the end of pBVD-6 was inserted in reading-frame at the EcoRI site of the bacterial lipoprotein gene in the plasmid vector pIM III (See Figure 4). A 1450 bp XbaI-SalI fragment from the BVD-pIM III construction was made blunt-ended by filling in with DNA polymerase Klenow fragment and cloned into the SmaI site of pGS20. The inserted fragment will encode an unmodified protein of approximately 18,000 Daltons. The construct utilizes both the bacterial initiation and termination codons and encodes a total of 23 bacterial amino acids; the remainder is BVDV encoded.

The remaining three BVD-pGS20 constructs involved the insertion of various BVDV gene fragments into established cDNA clones of equine influenza viral (EIV) surface antigen genes. In each case the BVDV fragments are inserted in reading-frame with the EIV genes such that a chimeric protein results. These constructs were designed to take advantage of eucaryotic membrane signal and anchor sequences present in the EIV hemagglutinin and neuraminidase genes. These genes have been independently inserted into recombinant vaccinia genomes and have been shown to be expressed in a functional (membrane-bound) fashion. Therefore, inclusion of the EIV gene signals in BVDV constructs will facilitate deposition of BVDV antigen on the surface of recombinant vaccinia infected cells. (2) The second BVD-pGS20 construct (Figure 5) utilized a PvuII (position #235 on Figure 1) to a FnuDII (position #698) BVDV fragment which is inserted into a BalI site of the equine EIV H3 hemagglutinin gene. The insertion involved blunt-end ligation of the fragment into the viral cDNA. (3) The third BVD-pGS20 construct (pGS20 H3BVD-3), Figure 6) employed a BVDV fragment spanning from the PvuII site (position #235) to the XhoII site (position #1026). This fragment is blunt-end inserted in reading frame into the EIV H3 hemagglutinin gene at HincII-BgII sites. (4) The fourth BVD-GS20 construct (pGS20 N7-BVD, Figure 7) extends from the EcoRI site (position #517) to the XhoII site (Blunt-ended by filling in with DNA polymerase Klenow fragment) (position #1026) of the BVD-27 clone. The BVDV fragment is inserted into the EIV neuraminidase N7 gene at EcoRI-StuI sites using the common EcoRI termini and blunt termini on the 5' and 3' ends, respectively, of both fragments. The XhoII-StuI ligation generated a BamHI site at the 3' end of the BVDV gene sequences and the pre-existing BamHI site at the 5' end of the N7 sequences was employed. The N7-BVD gene fusion was moved into the BamHI site of pKT1941.4, a plasmid which supplied a stop codon 18 nucleotides downstream from the 3' terminus of the BVDV sequences. The last three fusion genes were inserted into the BamHI or BamHI-SmaI sites of pGS20 (see Figures 5, 6 and 7).

## 2. Preparation of BVDV recombinant vaccinia viruses

The recombinant vectors described above are transfected into cells infected with wild-type vaccinia virus, which was received from Wyeth Laboratories, Inc. (Marietta, PA) and plaque purified twice in CV-1 cells. A small aliquot of virus stock (0.1 ml) is diluted with an equal volume of trypsin (0.25 mg/ml) and incubated for 30 min at 37°C with vortexing, followed by sonication to disperse any cell clumps. The virus is diluted to a concentration of 5x 10⁴ plaque-forming units per ml in phosphate-buffered saline (PBS) containing 0.2% bovine serum albumin (BSA) and penicillin/steptomycin. CV-1 cells are infected in monolayers on 60 mm plates with 1 ml vaccinia virus to give a multiplicity of infection of 0.05 pfu/cell. The virus inoculum is incubated on the cell for 2 hr at 37°C with rocking.

The DNA for transformation is prepared as described in Graham, et al., Virology (1973) 52:456; Stow, et al., J. Gen. Virol. (1976) 33:447; and Frost, et al., Virology (1978) 91:39. Briefly, 5-10 g of plasmid (pGS20 derivative) DNA, and 1-2μg wild-type vaccinia virus DNA are added to 1 ml to Hepes-buffered saline (0.14 M NaCl, 5 mM KCl, 1 mM Na phosphate, 0.1% dextrose, 20 mM Hepes, pH 7.05), and 50 μl of 2.5 molar CaCl₂ is added. The solutions are mixed and left at room temperature for 30 minutes and the desired precipitate of DNA forms in this time.

The virus inoculum is aspirated from the CV-1 cell monolayers; 1 ml of the DNA precipitate is substituted; and the monolayers left at room temperature for 30 min. after which 4 ml prewarmed Eagle's MEM containing 8% fetal bovine serum (FBS) is added. The monolayers are incubated for 3.5 hr at 37°C, before aspirating off the medium and replacing it with 5 ml fresh Eagle's MEM containing 8% FBS. The monolayers are then left at 37°C for 2 days, resulting in the development of vaccinia cytopathic effects. The cells and virus are harvested by scraping, pelleted, and resuspended in 0.5 ml Eagle's MEM, and frozen at -70°C

positive for antibodies to BVDV. The filters were washed extensively in Tris-saline, 3% BSA containing 1 X $10^6$ cpm/ml of $^{125}$I-labeled Staphylococcus aureus protein A. Following washing in Tris-saline, the filters were used to expose Kodak XAR-5 film at -70°C with an intensifying screen. Two positive clones (BVD-1 and BVD-6) identified by the antibody screen were isolated and shown by rescreening to breed true for expression of BVDV antigen.

## 2. Sequence of BVD Viral cDNA

The complete nucleotide sequences of the BVDV cDNA segment contained in clones BVD-1 and pBVD-6 were determined. Fragments generated by restriction endonuclease digestion of the pBVD inserts were cloned at various sites into the polylinker region of replicative forms of m13 mp8 and mp9. Single-stranded phage DNA was isolated and sequence determined by the dideoxy chain termination DNA sequencing procedure as described by Sanger, et al. Proc. Natl. Acad. Sci. USA (1977) 74:5463.

The BVDV sequence of pBVD-1 was found to be a subset of the pBVD-6 clone (see Figure 1). Each clone contains an open reading frame that can be translated in frame with the plasmid B-lactamase gene, as shown in Figure 1. Following expression of B-lactamase, one encounters 166 amino acids from the pBVD-6 reading frame and 11 out-of-frame amino acids. The B-lactamase/BVDV fusion polypeptide has a predicted length of 359 amino acids and an estimated molecular weight between 40,000 and 43,000 daltons.

The predicted B-lactamase-BVDV fusion polypeptide expressed by pBVD-6 is seen in $^{35}$S-labeled bacterial cell extracts immunoprecipitated with anti-BVDV serum or with anti-hog cholera virus serum. Most of the immunoprecipitable BVDV antigen labeled in steady state labeling of cells containing pBVD-6 is localized in the periplasmic space outside the bacterial cell membrane.

## 3. Isolation of Overlapping BVD Viral cDNA

The cDNA insert contained in pBVD-6 was utilized as a radioactive probe to screen the original BVDV cDNA library for overlapping, adjacent sequences contained in other clones. The pBVD-6 insert was radioactively labeled with $^{32}$p by the nick-translation method of Rigby, P.W.J., et al., J. Mol. Biol. (1977) 131:237-251. Replica filters onto which the BVDV cDNA library had been plated were made, as described above, then prepared for hybridization according to the method of Grunstein, M. and Hogness, D., Proc. Natl. Acad. Sci. USA (1975) 72:3961. The filters are prewashed in hybridization buffer (0.75M NaCl, 0.075 M NaCitrate, 50% formamide, 0.5% sodium dodecyl sulfate (SDS), 0.02% BSA, 0.02% Ficoll -400,000, 0.02% polyvinyl pyrollidone, 0.01% $Na_4P_2O_7$, 20 μg/ml denatured sheared salmon sperm DNA) at 42°C for 18 hours. Five X $10^5$ cpm of $^{32}$P-labeled boiled pBVD-6 cDNA was added per ml fresh hybridization buffer and the filters were incubated in this buffer at 42°C for 16 hours. They were then washed in 0.3 M NaCl, 0.03 M NaCitrate and 0.5% SDS two times, each for 30 minutes at 50°C, and exposed for autoradiography overnight. Two clones were positively identified, p-BVD4 and pBVD-19, and were sequenced in a manner identical to BVDV cDNA clones pBVD-1 and pBVD-6. Clone pBVD-19 was found to overlap at the 3'-terminus of pBVD-6 and extended the in-frame nucleotide sequence 3' by 450 base pairs. Clone pBVD-4 was found to overlap at the 5' terminus of pBVD-6 and extended the in-frame nucleotide sequence 5' by 400 base pairs. The nucleotide sequences of pBVD-1, pBVD-4, pBVD-6, and pBVD-19 taken together provides a continuous sequence of 1147 base pairs, which can be translated in one open reading frame - (Figure 1). This fused sequence representing pBVD-1, pBVD-4, pBVD-6, and pBVD-19 was designated pBVDV-27.

E. coli K12 strain MC1061 containing pBVD-27 was deposited with the American Type Culture Collection (ATCC) 12301 Parklawn Drive, Rockville, MD 20852 on February 21, 1986 and accorded the accession number 67004.

## 4. Specificity of BVDV Clones

The clones sequence in pBVD-1 (Figure 1) was shown to be BVDV specific by hybridization of the pBVD-1 insert to RNA isolated from BVDV infected cells.

Semi-confluent bovine testicle cells were infected with a 1:10 dilution of a stock of the Singer strain of BVDV (stock E204-4, titered at 9.3 logs by $TCID_{50}$). Medium was removed from cells and virus was added in 0.5 ml media. After one hour at 37°C, the cells were rinsed with media, then grown at 37°C under 10 ml media. A second flask of cells was mock-infected with media alone.

At 20 hours post-infection, cells were rinsed once with cold (4°C) phosphate-buffered saline (PBS), then removed from the flask by trypsinization and pelleted by low speed centrifugation. The cells were rinsed with cold PBS, resuspended in cold 0.1X RSB (1mM Tris-HCl, pH 7.5, 0.5 mM NaCl, 0.1 mM $MgCl_2$), allowed to swell 5 minutes on ice, then homogenized by ten strokes in a Dounce homogenizer. Cell nuclei were pelleted by low speed centrifugation. The cytoplasmic supernatant was made 1% SDS and 20 mM EDTA, and then extracted with a 1:1 mixture of chloroform and phenol. Cellular RNA was precipitated using 70% ethanol as described previously, and resuspended in 10 mM Tris-HCl, pH 7.5, 1 mM EDTA.

3.5 $\mu$g each of RNA from BVDV-infected and mock-infected cells was denatured in 10 mM methylmercuric hydroxide at 68°C for 3 minutes, and fractionated by electrophoresis in a 1.4% agarose gel containing 10 mM methylmercuric hydroxide by the method of Bailey, J.M. and Davidson, M., Anal. Biochem., (1976) 70:75-85. Following electrophoresis, the RNA was transferred to a nitrocellulose membrane in 20 X SCC (1 X SCC = 3.0 M NaCl, 0.3 M Na Citrate) overnight by the method of Thomas, P.S., Proc. Natl. Acad. Sci. USA, (1980) 87:5201.

Each nitrocellulose membrane was baked for 2 hours at 80°C in vacuo, then prehybridized in a solution containing 0.75 M NaCl,0.075 M Na Citrate, 50% formamide, 0.5% SDS, 0.02% BSA, 0.02% Ficoll-400,000, 0.02% polyvinyl pyrollidone, 50 $\mu$g/ml yeast t-RNA and 50 $\mu$g/ml denatured, sheared salmon sperm DNA at 42°C for 4 hours. The membranes were hybridized at 42°C for 18 hours in the above solution with gel-purified pBVD-1 insert which was radioactively labeled by nick-translation according to the procedure of Rigby, P.W.J., et al., J. Mol. Biol. 113:237-251 (1977). The blot was washed for 1 hour at 50°C in 0.2 X SCC and 0.1% SDS, then dried and autoradiographed on Kodak® XAR film with a Dupont® Cronex® intensifying screen overnight at -70°C.

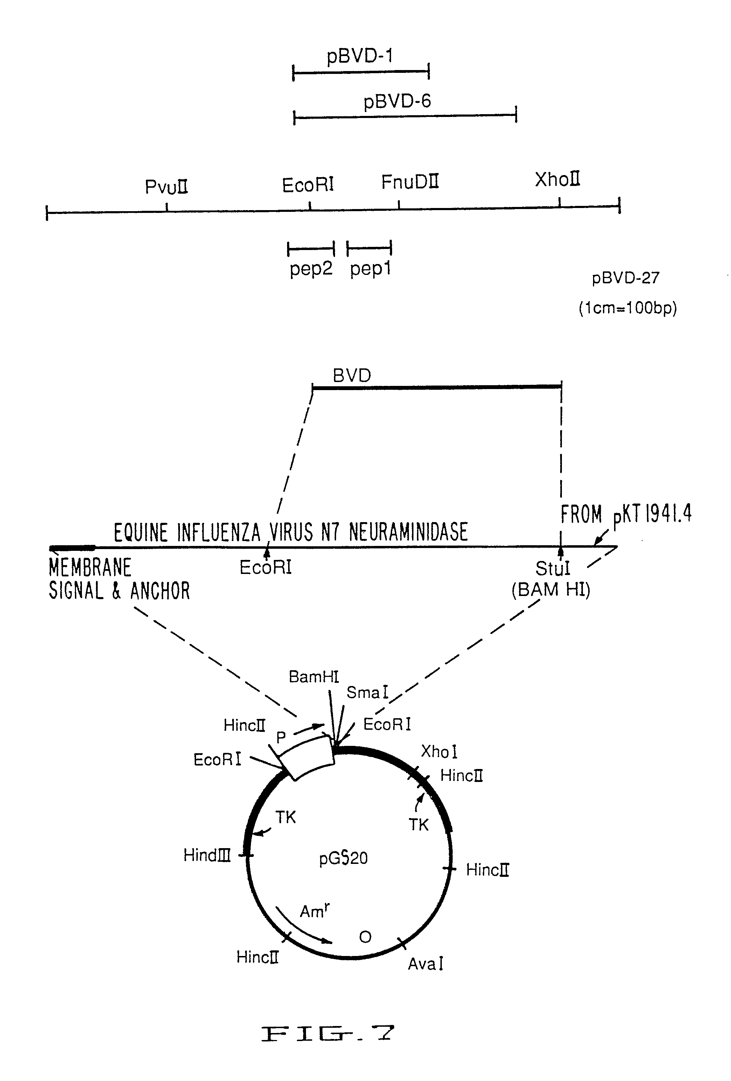

The pBVD-1 probe did not hybridize to mock-infected cell RNA, but shows positive hybridization to high-molecular weight RNA species of 10,000-12,000 nucleotides in BVDV infected cell RNA (Figure 2). This class of BVDV specific cellular RNAs has been previously reported by Purchio, A.F. et al. , J. Virol., 48:320:324 (1983).

### 5. Identification of BVD viral antigen corresponding to cDNAs

To determine which viral antigen of BVDV is encoded by the described set of BVDV cDNAs, labeled viral antigens were prepared. Bovine testicle cell monolayers were infected as described previously, using media containing $^{35}$S-methionine. The infected labeled cells were harvested by trypsinization and concentrated by pelleting. Mock-infected labeled cells were also prepared as described. The cells were lysed and the proteins denatured by resuspending the cell pellets in 2.5% SDS, 5% B-mercaptoethanol, 10% glycerol and 50 mM Tris-HCl, pH 6.8, at 100°C for five minutes. A sample of each lysate was used for immumoprecipitation with antibodies produced by synthetic peptides designed from the BVD-6 cDNA sequence (see Section D and Figure 1). Antibodies to BVDV synthetic peptide #2 (pep-2) recognized a BVDV-specific protein of 55,000 Daltons as characterized by polyacrylamide gel electorphoresis. BVDV synthetic peptide #1 (pep-1) antisera did not immunoprecipitate the 55,000 Dalton protein mock-infected cells. The 55,000 Dalton antigen has been previously identified and characterized as one of the major surface glycoproteins of the BVDV (Purchio, A.F., et al., supra) and is likely to elicit the production of a protective immune response in an infected host.

### B. Expression of BVDV Chimeric Proteins in Vaccinia Virus Vector

#### 1. Vector contructions:

Methods described by Mackett, et al.(supra), were used to insert BVDV cDNAs into vaccinia recombinants.

Each cDNA was first subcloned into pGS20 which contains the 7.5 K vaccinia gene promoter with unique BamHI and SmaI sites immediately downstream for the insertion of the desired heterologous gene, which is in turn flanked by vaccinia thymidine kinase (TK) gene. The TK-gene permits homologous recombination into the vaccinia genome and affords a method of selection (generation of tk-viruses).

The resuspended virus/cell pellets are freeze-thawed three times by freezing the cells at -70°C for 30 minutes and quickly thawing at 37°C, followed by sonication for one minute to disperse the virus/cell clumps. The resulting crude virus stock is then inoculated in serial 10-fold dilutions into 143 cells (Mackett, M. et al., J. Virol. (1984) 49 :857-864), a human tk-cell line. After two hours at 37°C, the 143 monolayers are then overlaid with 1% agarose containing 1x modified Eagle's medium, 5% FBS, and 25μg/ml 5-bromodeoxyuridine (BUdR). After incubation for two days at 37°C, BUdR-resistant plaques are picked and grown for 48 hours at 37°C in 24-well plates of 143 cell monolayers in the presence of 25 μg/ml BUdR.

Virus is harvested and assayed for the presence of the BVDV-derived genetic material by DNA-DNA dot blot hybridization, as follows: cells are scraped from the dish into a Eppendorf centrifuge tube, centrifuged for 1 minute, and the cell pellet resuspended in 0.2 ml PBS. After freeze-thawing 3 times and sonicating as described above, the sonicate is applied to a nitrocellulose filter and air dried. A wild-type virus control is also spotted onto the same filter. The filter is then placed on paper soaked in (1) 0.5 M NaOH, (2) 1 M Tris-HCl, pH 7.5, and (3) 2x SSC, for 5 minutes each. The filter is then baked at 80°C under vacuum for 2 hours. The baked filter is prehybridized for 1-4 hours at 42°C in 5 ml 50% formamide, 4x SSC, 5x Denhardt's solution, and 0.1 mg/ml sheared and boiled salmon sperm DNA. The filter is then hybridized overnight at 42°C to 2x $10^7$ cpm of $^{32}$P-labeled BVDV gene probe in 5 ml prehybridization buffer. The probe is prepared by isolating 5μg of BVDV gene insert from one of the pGS20-derivative vectors and nick translating 0.5-1 μg of the isolated DNA using a commercially available nick translation kit (Bethesda Research Labs). The filter is then washed twice for 30 minutes in 0.5x SSC, 0.1% SDS, air dried, and autoradiographed overnight at -70°C as previously described.

Virus containing BVDV gene inserts, as determined by dot blot hybridization, are infected onto monolayers of 143 cells and left until a confluent cytopathic effect is obtained. The culture medium is then aspirated off and cells lysed in 1% SDS, 0.1 M B-mercaptoethanol, 50 mM Tris-HCl, pH 7.8. The lysate is made 0.5 mg/ml is proteinase K, incubated 4 hours at 37°C, phenol extracted, ethanol precipitated, and the resultant DNA is analyzed by restriction enzyme Southern blot analysis to show that the vaccinia genomes indeed contain the desired BVDV genetic material. The fact that BVDV gene sequences have been successfully inserted in frame with equine influenza virus gene sequences is confirmed by the dideoxy chain termination method of DNA sequencing (see Examples 2.A.).

### 3. Expression of BVDV and BVDV-EIV Chimera Surface Antigens by Vaccinia Recombinants

BVDV recombinant constructs 3 & 4 (Figure 6 and Figure 7) are plaqued separately into CV-1 monolayers. The monolayers are washed, fixed, and incubated with bovine anti-BVDV sera. Antibody binding specifically to BVDV peptide antigens is detected by a subsequent incubation with $^{125}$I-labeled staphylococcal A protein followed by autoradiography. In each case, all recombinant virus plaques will bind the appropriate antibody.

Immunoprecipitation of $^{35}$S-labeled cellular lysate obtained from monolayers of CV-1 cells infected by individual vaccinia-BVDV recombinants showed the presence of BVDV chimeric proteins. In the case of these recombinant virus constructs, antisera raised against the BVDV-infected cells immunoprecipitated a protein of the predicted molecular weight which was identified by polyacrylamide gel electrophoresis and autoradiography.

E. coli K12 strain MC1061 containing pGS20-N7BVD was deposited with the American Type Culture Collection (ATCC) 12301 Parklawn Drive, Rockville, MD 20852 on February 21, 1986 and accorded the accession number 67005.

E. coli K12 strain MC1061 containing pGS20-H3BVD-3 was deposited with the American Type Culture Collection (ATCC) 12301 Parklawn Drive, Rockville, MD 20852 on February 21, 1986 and accorded the accession number 67006.

#### 4. Amplification of Vaccinia Recombinants

After the vaccina-BVDV recombinants are identified as both carrying the appropriate BVDV genetic segment and expressing the encoded BVDV protein, a single plaque of each is isolated twice in 143 cells in the presence of BUdR. The twice purified isolate is amplified once in 143 cells in the presence of BUdR and subsequently in CV-1 cells in the absence of selective pressure. The resulting vaccinia-BVDV recombinants are stable genetically and can be further amplified in CV-1 cells.

#### 5. Bioassay of Vaccinia Recombinants

Calves are used as subjects to assess the ability of the recombinant vaccinia to raise titers of neutralizing anti-BVDV antibodies in serum.

Pairs of calves are inoculated with wild-type or recombinant virus by intradermal administration of 1-2x $10^8$ plaque-forming units distributed in 2-3 sites on the back, and are bled at days 0, 14, and 28. The sera are then tested for the presence of anti-BVDV neutralizing antibodies.

The serum neutralization assay is performed by the following procedure carried out in a 96-well cell culture microtitre plate. $50\mu l$ of complete Eagle's MEM containing 5% lamb or horse serum is added to each well of the microtitre plate. $50\mu l$ of each serum sample to be tested is added to the first well of a row and serial two-fold dilutions out to 1:512 are made using a 50 $\mu l$ Dynatech Rotatitre Dilutor. Each serum sample is assayed in triplicate. 100 $TCID_{50}$ units of a BVDV viral stock which has been titered in the same system is added to each well of the microtitre plate. The plate is then incubated at 25°C for 1 hour after which approximately $10^4$ bovine testicle cells are added to each well. The microtitre plate is incubated for 5 days at 37°C at which time cytopathic effect of the cell monolayer is scored visually. A serum neutralization titre is the highest dilution of serum which completely protects the cell monolayer against the cytopathic effects of the virus.

#### C. Synthetic BVDV Peptides

#### 1. Chemical Peptide Synthesis

The peptides can be produced by the well known solid phase method as described (Merrifield, J. Amer. Chem. Sec. 85:2149-2154 (1963)). In the solid phase method of peptide synthesis, peptides of 3 to 40 amino acids in length (usually 10-25 residues) are synthesized with the peptide chain attached to beads of polystyrene resin. The first amino acid is attached to the resin followed by removal of the alpha-amino protecting with trifluoroacetic acid in methylenechloride. This deprotection is carried out at room temperature after removal of the alpha-amino protecting group. The next amino acid is added as a suitably protected derivative by activation and coupling of the carboxyl functionality.

After the desired amino acid sequence has been synthesized, the polypeptide is removed from the resin by treatment with liquid hydrogen fluoride, anisole and methylethylsulfide. The polypeptide is extracted from the resin and freeze dried. Purification of the crude peptide is accomplished by one or more of the purification techniques including gel filtration, position chromatography, ion-exchange chromatography, and preparative liquid chromatography.

The protecting groups used throughout the solid phase synthesis are well known and standard derivatives of amino acids. The alpha-amino protecting groups are of the aliphatic urethane protecting group class illustrated by tert-butyloxycarbonyl and tert-amyloxcarbonyl.

Protection for the phenolic hydroxy group of tyrosine may be benzyl, 2, 6-dichlorobenzyl, benzyloxycarbonyl or 2-romobenzyloxycarbonyl residues.

Protection from the sulfhydryl grou of cysteinyl residues is from either benzyl, 4-methylbenzyl, 3, 4-dimethylbenzyl, acetamidomethyl, trityl, 4-methoxybenzyl, etc.

Protection of the indole ring of tryptophan is by the formyl group, arginyl and histidyl residues by tosyl groups and lysyl side chains by benzyloxycarbonyl, toscyl or 2-chlorobenzyloxycarbonyl groups.

Synthetic peptides derived from the BVDV sequence were prepared using this methodology are are listed in the following section.

## 2. Description of BVDV synthetic peptides

The BVDV-specific deduced amino acid sequence of cDNA clones pBVDV-6 plus pBVDV-4 was analyzed using Robson secondary structure prediction method and the Kyte and Dolittle algorithm to predict sequence hydrophobicities. Two possible antigenic regions within the BVDV sequence were identified and synthetic peptides encompassing these regions were prepared (Figure 1). Pep-1 is a 27 amino acid peptide with the sequence:

S-P-E-M-K-L-E-D-T-M-V-I-A-W-C-K-D-G-K-F-T-Y-L-P-R-C-T.

The sequence is highly charged, containing some possible alpha-helical structure and a potential external loop. The sequence also contains a possible disulfide bride. Pep-2 is a 28-mer of the sequence:

I-T-G-V-Q-G-D-L-H-C-K-P-E-F-S-Y-A-I-A-R-D-E-R-I-G-Q-L-G

This charged sequence contains two possible external loops.

## 3. Preparation of synthetic peptides for immunization

Synthetic peptides were either self-polymerized with glutaraldehyde or covalently coupled to hemocyanin via 1-ethyl-3-(3-dimethylaminopropyl) carbodiimide. Self-polymerization with glutaraldehyde was performed at 20°C for 2 hours in a reaction mixture containing 4-10 mg/ml peptide, 50 mM NaHPO$_4$ (pH 7.5) and 150 mM glutarldehyde. Under these conditions, self-polymerization resulted in insoluble products after 1 hour. This material was resuspended and mixed under appropriate conditions with Freund's adjuvant for immunization. Covalent coupling of peptides to hemocyanin was performed in a two step procedure. First, peptide (4-10 mg/ml) was incubated at 40°C for 20 min. in the presence of 1 mg/ml carbodiimide in distilled water adjusted to pH 3.5 with 1 N HCl. Second, hemocyanin was added (1 mg/ml) and the pH of the solution was adjusted to pH 9.0 with five volumes of distilled water containing 1 mM NaOH. This mixture was incubated for 2 hours at 21°C and dialyzed against phosphate buffered saline at 4°C for 16 hours. This procedure effectively couples the carboxy terxinus of the peptides to free amino groups present in the hemocyanin. After dialysis, the conjugated peptides were mixed in appropriate amounts with Freund's adjuvant and used for immunization.

## 4. Immunization of rabbits with synthetic peptides

Both peptides were used to raise anti-peptide and anti-BVDV protein antibodies in rabbits. Groups of two rabbits each were inoculated with either peptide in one of two forms: free peptide or conjugated to KLH carrier. The primary inoculation was followed by boosts at three weeks and at six weeks. Preimmune and immune sera from immunized and control animals were analyzed for the ability to immunoprecipitate $^{35}$S-methionine-labeled proteins from BVDV-infected cells.

Rabbit antisera raised against Pep-2 specifically immunoprecipitated the 55,000 Dalton glycosylated surface antigen of BVDV described earlier which is known to be a major viral surface protein also common to hog cholera virus. This reiterates the highly immunogenic nature of the original BVDV cDNA clones isolated.

## 5. Immunization of cows with synthetic peptides

Four angus x holstein crossed BVDV-negative calves (pathogen-free containment from birth and monitored serologically) were used in an experiment to test the ability of the BVDV synthetic peptides to prime animals for subsequent innoculation with whole killed BVDV vaccine. Animals A and B received two injections of 500μg each of conjugated BVDV Pep-2 at three week intervals. Animal C received two injections of 500 μg each of conjugated BVDV Pep-1 at three week intervals. Animal D received no injections. All four animals received a single dose (5 mls) of Ft Dodge killed BVDV vaccine at six weeks after the final peptide inoculation. All animals were bled at one, two, three, four and five weeks post Ft Dodge vaccination and sera were assayed by the BVDV-sera neutralization test as described previously. The results of the assay are presented in Table I and indicate that cows that received a priming inoculation of peptides 1 and 2 mount an earlier serum neutralization response that the animal receiving no peptide primer.

## Table I

### Effect of BVD Synthetic Peptides
### as Primes for Killed Whole Virus Immunizations

| Cow | Peptide Inoculations | Weeks Post Fort Dodge Vaccine Immunization | | | | |
|---|---|---|---|---|---|---|
| Angus x Holst | | 1 | 2 | 3 | 4 | 5 |
| Rm5 | pep2 x 2 | 0 | 5.6 | 6.7 | 6.7 | 19 |
| | | 0 | 11 | 9.5 | 11 | 19 |
| Rm6 | pep2 x 2 | 0 | 3.4 | 3.4 | 9.5 | 38 |
| | | 0 | 5.6 | 4.7 | 19 | 38 |
| Rm8 | pep1 x 2 | 0 | 4.7 | 9.5 | 22 | 29 |
| | | 0 | 0 | 27 | 22 | 27 |
| Rm7 | none | 0. | 0 | 0 | 4.7 | 9.5 |
| | | 0 | 0 | 0 | 9.5 | 12 |

In addition, at five weeks post Ft Dodge vaccination, peptide-primed calves exhibit a two to four fold higher serum neutralization titre as compared to the untreated animal.

These results indicate that BVDV-specific synthetic peptides can be used to potentiate the serum neutralization activity induced by a commercial killed BVDV vaccine.

Although the foregoing invention has been described in some detail by way of clarity and for purposes of understanding, it will be understood by those skilled in the art that modifications of the invention may be practiced while remaining within the spirit and scope of the appended claims.

**Claims**

1. An immunogenic peptide comprising a sequence of amino acid residues which is capable, when introduced in an immunogenically effective amount into a host, of inducing the host to produce antibodies which recognize and specifically bind to at least one member selected from the group consisting of virus of the family Togaviridae, the Togavirus, a Togavirus protein, the immunogenic peptide, and immunogenically related variants thereof.

2. An immunogenic peptide as recited in claim 1 comprising a peptide selected from the group of peptides in accordance with any of the following formulae:

I. $X_1$-$AA_1$-$AA_2$-$AA_3$-$AA_4$-$AA_5$-$AA_6$-$AA_7$-$AA_8$-$AA_9$-$AA_{10}$-$AA_{11}$-$AA_{12}$-$AA_{13}$-$AA_{14}$-$AA_{15}$-$AA_{16}$-$AA_{17}$-$AA_{18}$-$AA_{19}$-$AA_{20}$-$AA_{21}$-$AA_{22}$-$AA_{23}$-$AA_{24}$-$AA_{25}$-$AA_{26}$-$AA_{27}$-$Y$, wherein:

$AA_1$ is any neutral, polar amino acid residue;

$AA_2$ and $AA_{24}$ are each independently any neutral, non-polar amino acid residue;

$AA_3$ and $AA_7$ are each independently any acidic, polar amino acid residue;

$AA_4$ and $AA_{10}$ are each independently any neutral, non-polar amino acid residue;

$AA_5$, $AA_{16}$ and $AA_{19}$ are each independently any basic, polar amino acid residue;

$AA_6$ and $AA_{23}$ are each independently any neutral, non-polar amino acid residue;

$AA_8$ and $AA_{17}$ are each independently any acidic, polar amino acid residue;

$AA_9$, $AA_{21}$ and $AA_{27}$ are each independently any neutral, polar amino acid residue;

$AA_{11}$ is any neutral, non-polar amino acid residue;

$AA_{12}$ is any neutral, non-polar amino acid residue;

$AA_{13}$ is any neutral, non-polar amino acid residue;

$AA_{14}$ is any neutral, non-polar amino acid residue;

$AA_{15}$ and $AA_{26}$ are each any neutral, polar amino acid residue, preferably C;

$AA_{18}$ is any neutral, non-polar amino acid residue, preferably G;

$AA_{20}$ is any neutral, non-polar amino acid residue, preferably F;

$AA_{22}$ is any neutral, polar amino acid residue, preferably Y; and

$AA_{25}$ is any basic, polar amino acid residue, preferably R;

22

and wherein:

$X_1$ is hydrogen, amido, acetyl, or additionally includes an amino acid, dipeptide, tripeptide or an oligopeptide of up to 200 amino acid residues, particularly the sequence of residues disclosed as residues 1 to 197 in Figure 1, and including N-acetyl derivatives thereof;

$Y_1$ is hydroxyl, amido, or an amino acid, dipeptide, tripeptide or an oligopeptide of up to 160 amino acid residues, particularly the sequence of residues disclosed as residues 227 to 383 in Figure 1, including Carboxy-terminal amide derivatives thereof.

$X_2$-$aa_1$-$aa_2$-$aa_3$-$aa_4$-$aa_5$-$aa_6$-$aa_7$-$aa_8$-$aa_9$-$aa_{10}$-$aa_{11}$-$aa_{12}$-$aa_{13}$-$aa_{14}$-$aa_{14}$-$aa_{15}$-$aa_{16}$-$aa_{17}$-$aa_{18}$-$aa_{19}$-$aa_{20}$-$aa_{21}$-$aa_{22}$-$aa_{23}$-$aa_{24}$-$aa_{25}$-$aa_{26}$-$aa_{27}$-$aa_{28}$-$Y_2$

wherein:

$aa_1$, $aa_{18}$ and $aa_{24}$ are each any neutral, non-polar amino acid residue, preferably I;

$aa_2$ is any neutral, polar amino acid residue, preferably T;

$aa_3$, $aa_6$ $aa_{25}$ and $aa_{28}$ are each independently any neutral, polar amino acid residue;

$aa_4$ is any neutral, non-polar amino acid residue;

$aa_5$ and $aa_{26}$ are each independently any neutral, polar amino acid residue;

$aa_7$ and $aa_{21}$ are each independently any acidic, polar amino acid residue;

$aa_8$ and $aa_{27}$ are each independently any neutral, non-polar amino acid residue;

$aa_9$ is any basic, polar amino acid residue;

$aa_{10}$ is any neutral, polar amino acid residue;

$AA_{11}$ is any basic, polar amino acid residue;

$aa_{12}$ is any neutral, non-polar amino acid residue;

$aa_{13}$ and $aa_{22}$ are each independently any acidic, polar amino acid residue;

$aa_{14}$ is any neutral, non-polar amino acid residue;

$AA_{15}$ is any neutral, polar amino acid residue;

$aa_{16}$ is any neutral, polar amino acid residue;

$aa_{17}$ and $aa_{19}$ are each independently any neutral, non-polar amino acid residue;

and

$aa_{20}$ and $aa_{23}$ are each independently any basic, polar amino acid residue;

and wherein:

$X_2$ is hydrogen, amido, acetyl, or additionally includes an amino acid, dipeptide, tripeptide or an oligopeptide of up to 160 amino residues, and including N-acetyl derivatives thereof;

$Y_2$ is hydroxyl, amido, or an amino acid, dipeptide, tripeptide or an oligopeptide of up to 200 amino acid residues, including Carboxy-terminal amide derivatives thereof;

(IA) $X_{1A}$-$AA_{15}$-$AA_{16}$-$AA_{17}$-$AA_{18}$-$AA_{19}$-$AA_{20}$-$AA_{21}$-$AA_{22}$-$AA_{23}$-$AA_{24}$-$AA_{25}$-$AA_{26}$-$Y_{1A}$

wherein:

$AA_{15}$ and $AA_{26}$ are each independently any neutral, polar amino acid residue;

$AA_{16}$ and $AA_{19}$ are each independently any basic, polar amino acid residue;

$AA_{17}$ is any acidic, polar amino acid residue;

$AA_{18}$ is any neutral, non-polar amino acid residue;

$AA_{20}$ is any neutral, non-polar amino acid residue;

$AA_{21}$ is any neutral, polar amino acid residue;

$AA_{22}$ is any neutral, polar amino acid residue;

$AA_{23}$ is any neutral, non-polar amino acid residue;

$AA_{24}$ is any neutral, non-polar amino acid residue; and

$AA_{25}$ is any basic, polar amino acid residue;

and wherein:

$X_{1A}$ is hydrogen, amido, acetyl, or additionally includes an amino acid, dipeptide, tripeptide or an oligopeptide of up to 220 amino acid residues, and including N-acetyl derivatives thereof; and

$Y_{1A}$ is hydroxyl, amido, or an amino acid, dipeptide, tripeptide or an oligopeptide of up to 160 amino acid residues, including Carboxy-terminal amide derivatives thereof;

(IIA) $X_{2A}$-$aa_3$-$aa_4$-$aa_5$-$aa_6$-$aa_7$-$aa_8$-$aa_9$-$aa_{10}$-$aa_{11}$-$Y_{2A}$

wherein:

$aa_3$ and $aa_6$ are each independently any neutral, polar amino acid residue;

$aa_4$ is any neutral, non-polar amino acid residue;

$aa_5$ is any neutral, polar amino acid residue;

$aa_7$ is any acidic, polar amino acid residue;

$aa_8$ is any neutral, non-polar amino acid residue;

$aa_9$ is any basic, polar amino acid residue;

23

$aa_{10}$ is any neutral, polar amino acid residue, and

$AA_{11}$ is any basic, polar amino acid residue;

and wherein:

$X_{2A}$ is hydrogen, amido, acetyl, or additionally includes an amino acid, dipeptide, tripeptide or an oligopeptide of up to 165 amino acid residues, and including N-acetyl derivatives thereof; and

$Y_{2A}$ is hydroxyl, amido, or an amino acid, dipeptide, tripeptide or an oligopeptide of up to 220 amino acid residues, including Carboxy-terminal amide derivatives thereof;

(IIB) $X_{2B}$-$aa_{20}$-$aa_{21}$-$aa_{24}$-$aa_{25}$-$aa_{26}$-$aa_{27}$-$aa_{28}$-$Y_{2B}$

wherein:

$aa_{20}$ and $aa_{22}$ are each independently any basic, polar amino acid residue;

$aa_{21}$ is any acidic, polar amino acid residue;

$aa_{22}$ is any acidic, polar amino acid residue;

$aa_{24}$ is any neutral, non-polar amino acid residue;

$aa_{25}$ and $aa_{28}$ are each independently any neutral, polar amino acid residue;

$aa_{26}$ is any neutral, polar amino acid residue; and

$aa_{27}$ is any neutral, non-polar amino acid residue;

and wherein:

$X_{2B}$ is hydrogen, amido, acetyl, or additionally includes an amino acid, dipeptide, tripeptide or an oligopeptide of up to 180 amino acid residues, and including N-acetyl derivatives thereof; $Y_{2B}$ is hydroxyl, amido, or an amino acid, dipeptide, tripeptide or an oligopeptide of up to 200 amino acid residues, including Carboxy-terminal amide derivatives thereof;

together with immunogenic bonded-monomeric-unit polymers or co-polymers, wherein at least one monomeric unit is such a peptide, and such immunogenic peptide bonded to immunogenic or non-immunogenic carrier material.

3. An immunogenic peptide as recited in claim 2 comprising a peptide selected from the group of peptides in accordance with any of the following formulae:

$X_{1A}$-C-K-D-G-K-F-T-Y-L-P-R-C-Y-$_{1B}$;

wherein $X_{1A}$ and $Y_{1A}$ are as defined in claim 2;

$X_{2A}$-G-V-Q-G-D-L-H-C-K-$Y_{2A}$

wherein $X_{2A}$, and $Y_{2A}$ are as defined in claim 2;

$X_{2B}$-R-D-E-R-I-G-Q-L-G-$Y_{2B}$

wherein $X_{2B}$ and $Y_{2B}$ are as defined in claim 2;

I-T-G-V-Q-G-D-L-H-C-K-P-E-F-S-Y-A-I-A-R-D-E-R-I-G-Q-L-G; and

S-P-E-M-K-L-E-D-T-M-V-I-A-W-C-K-D-G-K-F-T-Y-L-P-R-C-T;

together with immunogenic bonded-monomeric-unit polymers or co-polymers, wherein at least one monomeric unit is such a peptide, and such immunogenic peptide bonded to immunogenic or non-immunogenic carrier material.

4. A vaccine capable of immunizing a host against infection by Togavirus comprising an immunogenically effective amount of at least one peptide in accordance with claim 2, together with a medium physiologically compatible with said host; said vaccine, when introduced to said host, being capable of stimulating the production of antibodies which specifically recognize and bind to said Togavirus.

5. A vaccine effective against BVDV virus or Hog cholera virus in bovine or swine hosts which comprises an immunologically effective portion of the amino acid sequence of a peptide in accordance with claim 2, bound to vaccinia virus, together with a physiologically acceptable carrier.

6. A method of immunizing cows or hogs against BVDV or hog cholera virus which comprises administering the vaccine of claim 4.

7. An immunogenic peptide as recited in claim 2 further comprising a bound label selected from the group consisting of radioactive tracers, fluorescers, and enzymes.

8. Antisera and antibodies which are capable of recognizing and specifically binding to an immunogenic peptide comprising the peptide in accordance with claim 2.

9. A DNA sequence comprising a sequence capable of directing the synthesis of a peptide in accordance with claim 2.

10. A DNA sequence comprising a sequence capable of directing the synthesis of the peptide a peptide in accordance with claim 2 bound to vaccinia virus.

11. An expression vector capable in a transformant microorganism or cell culture, of expressing a DNA sequence in accordance with claim 9 or 10.

12. A microorganism or cell culture transformed with the expression vector in claim 11, together with the transforming DNA sequences.

24

13. A microorganism or cell culture in accordance with claim 12 wherein said microorganism or cell culture is selected from the group consisting of cultures identified by ATCC Accession Numbers 67004, 67005 and 67006.

14. A process for producing an immunogenic peptide, which process comprises:

a) preparing an expression vector capable of expressing the DNA sequence of claim 9 or 10 in a host cell;

b) transforming a host cell culture with said expression vector;

c) culturing said transformed host cells under conditions permitting expression of said DNA sequence; and

d) recovering the expressed immunogenic peptide.

15. A process for producing an immunogenic peptide, which process comprises

a. preparing a protected peptide bonded to a solid carrier in a reaction mixture, wherein the peptide has an amino acid sequence in accordance with claim 2;

b. separating the solid carrier from the peptide;

c. deprotecting the peptide; and

d. isolating the peptide from the reaction mixture.

16. A diagnostic assay for detecting the presence of a togavirus antigen in a sample suspected of containing said antigen comprising the antibodies or antisera of claim 8 together with a means for determining specific immunoreaction when said antibodies or antisera react with said sample.

pBVD-27

50                                                                                              100

AAA ATT GTC GGC CCT GGG AAG TTT GAC ACC AAC GCA GAG GAC GGC AAG ATA CTA CAT GAG ATG GGG GGC CAC TTG TCG GAG GTA CTA CTA CTT TCT TTA GTG GTG CTG
Lys Ile Val Gly Pro Gly Lys Phe Asp Thr Asn Ala Glu Asp Gly Lys Ile Leu His Glu MET Gly Gly His Leu Ser Glu Val Leu Leu Leu Ser Leu Val Val Leu
                                                                                                                                    30

150                                                                                             200

TCC GAC TTT GCA CCG GAA ACA GCC AGC GCA ATG TAC CTA ATC CTA CAT TTT TCC ATC CCA CAA AGT GAC GTT GAC ATA ATG GAA TGT GAT AAG ACC CAG TTG AAC CTC
Ser Asp Phe Ala Pro Glu Thr Ala Ser Ala MET Tyr Leu Ile Leu His Phe Ser Ile Pro Gln Ser His Val Asp Ile MET Glu Cys Asp Lys Thr Gln Leu Asn Leu
                                                                                            60

            PvuII                      250                                                           300

ACA GTG GAG CTT ACA ACA GCT GAT GTA ATA CCA GGA TCA GTC TGG AAC CTA GGC AAA TGG GTA TGT ATA AGA CCA AAT TGG TGG CCT TAT GAG ACA ACT GTG GTG TTG
Thr Val Glu Leu Thr Thr Ala Asp Val Ile Pro Gly Ser Val Trp Asn Leu Gly Lys Trp Val Cys Ile Arg Pro Asn Trp Trp Pro Tyr Glu Thr Thr Val Val Leu
                                                            90

            350                                                                   400

GCA TTT GAA GAG GTG AGC CAG GTG GTG AAG TTA GTG TTG AGG GCA CTC AGA GAT TTG ACA CGC ATT TGG AAC GCT GCA ACA ACT ACT GCT TTC TTA ATA TGC CTT GTT
Ala Phe Glu Glu Val Ser Gln Val Val Lys Leu Val Leu Arg Ala Leu Arg Asp Leu Thr Arg Ile Trp Asn Ala Ala Thr Thr Thr Ala Phe Leu Ile Cys Leu Val
                    120

        450                                                      pBVD-6                               EcoRI

AAG ATA GTC AGG GGC CAG ATG GTA CAG GGC ATT CTG TGG CTA CTA CTG ATA ACA GGG GTA CAA GGG GAC TTG CAT TGC AAA CCT GAA TTC TCA TAT GCC ATA GCA AGG
Lys Ile Val Arg Gly Gln MET Val Gln Gly Ile Leu Trp Leu Leu Leu Ile Thr Gly Val Gln Gly Asp Leu His Cys Lys Pro Glu Phe Ser Tyr Ala Ile Ala Arg
            150                                    Pep-2 (I   T   G   V   Q   G   D   L   H   C   K   P   E   F   S   Y   A   I   A   R

    550                                                          600

GAT GAA AGA ATT GGT CAA CTG GGG GCT GAA GGC CTT ACT ACC ACT TGG AAG GAT TAC TCG CCT GAA ATG AAA CTG GAA GAC ACA ATG GTC ATA GCT TGG TGC AAA GAT
Asp Glu Arg Ile Gly Gln Leu Gly Ala Glu Gly Leu Thr Thr Thr Trp Lys Asp Tyr Ser Pro Glu MET Lys Leu Glu Asp Thr MET Val Ile Ala Trp Cys Lys Asp
D   E   R   I   G   Q   L   G)                          Pep-1 (S   P   E   M   K   L   E   D   T   M   V   I   A   W   C   K   D

650                                         EnuDII                                                  750

GGT AAG TTT ACG TAC CTC CCA AGG TGC ACG AGA GAA ACC AGA TAT CTC GCG ATC TTG CAT ACA AGA GCC TTA CCG ACC AGT GTG GTA TTC AAA AAA CTT TTT GAT GGG
Gly Lys Phe Thr Tyr Leu Pro Arg Cys Thr Arg Glu Thr Arg Tyr Leu Ala Ile Leu His Thr Arg Ala Leu Pro Thr Ser Val Val Phe Lys Lys Leu Phe Asp Gly
G   K   F   T   Y   L   P   R   C   T)                                                       240

                                    800                 850                                         900

CGA AAG CAA GAG GAT GTA GTC GAA ATG GAC GAC AAC TTT GAA TTT GGA CTC TGC CCA TGT GAT GCC AAA CCC ATA GTA AGA GGG AAA TTC AAT ACA ACG CTG CTG AAC
Arg Lys Gln Glu Asp Val Val Glu MET Asp Asp Asn Phe Glu Phe Gly Leu Cys Pro Cys Asp Ala Lys Pro Ile Val Arg Gly Lys Phe Asn Thr Thr Leu Leu Asn
                                                                        270

                                                            pBVD-6

GGA CCG GCC TTC CAG ATG GTA TGC CCC ATA GGA TGG ACA GGG ACT GTA AGC TGT ATG TCA TTC AAT ATG GAC ACC TTA GCC ACA ACC GTG ATA CGG ACA TAT AGA AGG
Gly Pro Ala Phe Gln MET Val Cys Pro Ile Gly Trp Thr Gly Thr Val Ser Cys MET Ser Phe Asn MET Asp Thr Leu Ala Thr Thr Val Ile Arg Thr Tyr Arg Arg
                                300

                    1000                                XhoII                       1050

TCC AAA CCA TTT CCT CAT AGG CAA GGC TGT ATC ACC CAA AAG ACT CTG GGG GAG GAT CTC CAT AAC TGC ATC CTC GGA GGA AAT TGG ACT TGT GTG CCT GGA GAC ATG
Ser Lys Pro Phe Pro His Arg Gln Gly Cys Ile Thr Gln Lys Thr Leu Gly Glu Asp Leu His Asn Cys Ile Leu Gly Gly Asn Trp Thr Cys Val Pro Gly Asp MET
                    330                                                                                                             360

            1100                                1149

CTA TTA TAC AAA GGG GGC TCT ATT GAA TCC TGC AAG TGG TGT GGT TAT CAA TTC TTT CAT CCA AGC TCC
Leu Leu Tyr Lys Gly Gly Ser Ile Glu Ser Cys Lys Trp Cys Gly Tyr Gln Phe Phe His Pro Ser Ser
                                                                            383

# FIG-1

FIG_2

0 236 977

FIG.3

0 236 977

FIG.4

FIG.5

FIG.7

FIG.6